# EUROPÄISCHE PATENTSCHRIFT

(11) **EP 1 664 058 B1**
(45) Veröffentlichungstag und Bekanntmachung des Hinweises auf die Patenterteilung: **14.11.2007**
(21) Anmeldenummer: 04764791.2
(22) Anmeldetag: 03.09.2004
(51) Int. Cl.: C07D 495/04, A61K 31/4188, A61P 1/00, A61P 11/00

(54) **SUBSTITUIERTE THIENOIMIDAZOLE, VERFAHREN ZU IHRER HERSTELLUNG, IHRE VERWENDUNG ALS MEDIKAMENT ODER DIAGNOSTIKUM SOWIE SIE ENTHALTENDE MEDIKAMENTE**
SUBSTITUTED THIENOIMIDAZOLES, METHOD FOR PRODUCTION AND USE THEREOF AS MEDICAMENT OR DIAGNOSTIC AND MEDICAMENTS COMPRISING THE SAME
THIENOIMIDAZOLES SUBSTITUES, PROCEDE DE PRODUCTION, UTILISATION COMME MEDICAMENT OU PRODUIT DE DIAGNOSTIC ET MEDICAMENTS CONTENANT CES COMPOSES

(30) Priorität: 08.09.2003 DE 10341240
(43) Veröffentlichungstag der Anmeldung: 07.06.2006
(73) Patentinhaber: Sanofi-Aventis Deutschland GmbH, 65929 Frankfurt am Main (DE)
(72) Erfinder: LANG, Hans-Jochen, 65719 Hofheim (DE); HEINELT, Uwe, 65187 Wiesbaden (DE); WIRTH, Klaus, 65830 Kriftel (DE); LICHER, Thomas, 65812 Bad Soden (DE)
(86) Internationale Anmeldenummer: PCT/EP2004/009836
(87) Internationale Veröffentlichungsnummer: WO 2005/026173

(56) Entgegenhaltungen:
- WO-A-02/46169
- JEN T ET AL: "AMIDINES AND RELATED COMPOUNDS. 6 STUDIES ON STRUCTURE-ACTIVITY RELATIONSHIPS OF ANTIHYPERTENSIVE AND ANTISECRETORY AGENTS RELATED TO CLONIDINE" JOURNAL OF MEDICINAL CHEMISTRY, AMERICAN CHEMICAL SOCIETY. WASHINGTON, US, Bd. 18, Nr. 1, 1975, Seiten 90-99, XP000567062 ISSN: 0022-2623

## Beschreibung

Die Erfindung betrifft substituierte Thienoimidazole. Medikamente, die Verbindungen dieses Typs enthalten, sind nützlich bei der Prävention oder Behandlung diverser Erkrankungen. So lassen sich die Verbindungen unter anderem verwenden zur Behandlung von Atemstörungen und des Schnarchens sowie zur Verbesserung des Atemantriebes, zur Behandlung von akuten und chronischen Erkrankungen der Niere und des Darms, durch ischämische und/oder Reperfusionsereignisse sowie durch proliferative oder durch fibrotische Ereignisse ausgelösten Erkrankungen, zur Behandlung oder Prophylaxe von Erkrankungen des zentralen Nervensystems, des Fettstoffwechsels und des Diabetes, der Blutgerinnung und des Befalls durch Parasiten.

Die Erfindung betrifft Verbindungen der Formel I oder II, 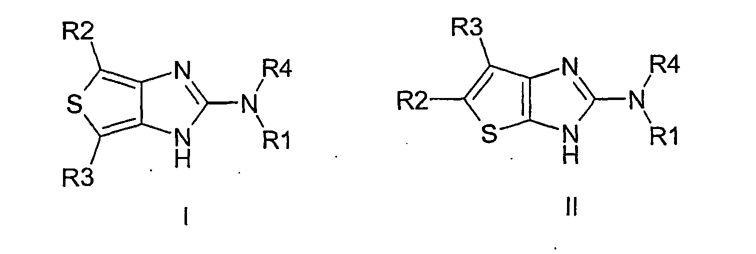 worin bedeuten
- R1: Phenyl, das in 2- und 6-Stellung mit den Resten R5 und R6 substituiert ist;
R5 und R6 unabhängig voneinander Wasserstoff, Methyl, Ethyl, Cycloalkyl mit 3, 4 oder 5 C-Atomen, Vinyl, Ethinyl, Fluor, Chlor, Brom, lod, Cyano, Trifluormethyl, SF₅, Methoxy, Nitro oder -X-R7;
R7 Alkyl mit 1, 2 oder 3 C-Atomen, Trifluormethyl oder CH₂-CF₃;
X CH₂, O, NH oder S(O)ₙ;
n Null, eins oder zwei; wobei R5 und R6 nicht beide gleichzeitig Wasserstoff entsprechen; oder
- R1: 3-Thienyl das in 2- und 4-Stellung mit den Resten R5 und R6 substituiert ist;
R5 und R6 unabhängig voneinander Wasserstoff, Methyl, Ethyl, Cycloalkyl mit 3, 4 oder 5 C-Atomen, Vinyl, Ethinyl, Fluor, Chlor, Brom, lod, CN, NO₂ Trifluormethyl, SF₅, Methoxy oder -X-R7;
R7 Alkyl mit 1, 2 oder 3 C-Atomen, Trifluormethyl oder CH₂-CF₃;
X CH₂, O, NH, S(O)ₙ;
n Null, eins oder zwei; wobei R5 und R6 nicht beide gleichzeitig Wasserstoff entsprechen;
- R2 und R3: unabhängig voneinander Wasserstoff, Fluor, Chlor, Brom, Methyl, CN, OH, -O- CH₃, NO₂, NH₂, -CF₃, oder -Y-R8;
R8 Methyl, Trifluormethyl oder CH₂-CF₃;
Y CH₂, O, NH oder S(O)ₘ;
m Null, eins oder zwei,
- R4: Wasserstoff, Methyl, Ethyl, Cyclopropyl, Cyclopropylmethyl oder CH₂-CF₃;
sowie deren pharmazeutisch verträgliche Salze und deren Trifluoressigsäuresalze.

Bevorzugt sind Verbindungen der Formel I,
worin bedeuten
- R1: Phenyl, das in 2- und 6-Stellung mit den Resten R5 und R6 substituiert ist;
R5 und R6 unabhängig voneinander Wasserstoff, Methyl, Ethyl, Cycloalkyl mit 3, 4 oder 5 C-Atomen, Vinyl, Ethinyl, Fluor, Chlor, Brom, lod, Cyano, Trifluormethyl, SF₅, Methoxy, Nitro oder -X-R7;
R7 Alkyl mit 1, 2 oder 3 C-Atomen, Trifluormethyl oder CH₂-CF₃;
X CH₂, O, NH oder S(O)ₙ;
n Null, eins oder zwei; wobei R5 und R6 nicht beide gleichzeitig Wasserstoff entsprechen;
oder
- R1: 3-Thienyl das in 2- und 4-Stellung mit den Resten R5 und R6 substituiert ist;
R5 und R6 unabhängig voneinander Wasserstoff, Methyl, Ethyl, Cycloalkyl mit 3, 4 oder 5 C-Atomen, Vinyl, Ethinyl, Fluor, Chlor, Brom, lod, CN, NO₂, Trifluormethyl, SF₅, Methoxy oder -X-R7;
R7 Alkyl mit 1, 2 oder 3 C-Atomen, Trifluormethyl oder CH₂-CF₃;
X CH₂, O, NH, S(O)ₙ;
n Null, eins oder zwei; wobei R5 und R6 nicht beide gleichzeitig Wasserstoff entsprechen;
- R2 und R3: unabhängig voneinander Wasserstoff, Fluor, Chlor, Brom, Methyl, CN, OH, -O- CH₃, NO₂, NH₂, -CF₃, oder-Y-R8;
R8 Methyl, Trifluormethyl oder CH₂-CF₃;
Y CH₂, O, NH oder S(O)ₘ;
m Null, eins oder zwei,
- R4: Wasserstoff, Methyl, Ethyl, Cyclopropyl, Cyclopropylmethyl oder CH₂-CF₃;
sowie deren pharmazeutisch verträgliche Salze und deren Trifluoressigsäuresalze.

Besonders bevorzugt sind Verbindungen der Formel 1,
worin bedeuten:
- R1: Phenyl, das in 2- und 6-Stellung mit den Resten R5 und R6 substituiert ist;
R5 und R6 unabhängig voneinander Wasserstoff, Methyl, Ethyl, Fluor, Chlor, Brom oder Trifluormethyl: wobei R5 und R6 nicht beide gleichzeitig Wasserstoff entsprechen;
oder
- R1: 3-Thienyl das in 2- und 4-Stellung mit den Resten R5 und R6 substituiert ist;
R5 und R6 unabhängig voneinander Wasserstoff, Methyl, Ethyl, Fluor, Chlor, Brom oder Trifluormethyl; wobei R5 und R6 nicht beide gleichzeitig Wasserstoff entsprechen;
- R2, R3 und R4: Wasserstoff;
sowie deren pharmazeutisch verträgliche Salze und deren Trifluoressigsäuresalze.

Eine Ausführungsform betrifft Verbindungen der Formeln I und II, in denen R5 und R6 unabhängig voneinander durch Wasserstoff, Methyl, Ethyl, Fluor, Chlor, Brom oder Trifluormethyl beschrieben werden, wobei R5 und R6 nicht beide gleichzeitig Wasserstoff entsprechen, bevorzugt sind Verbindungen, in denen R5 und R6 unabhängig voneinander durch Wasserstoff, Methyl, Fluor, Chlor oder Trifluormethyl beschrieben werden, wobei R5 und R6 nicht beide gleichzeitig Wasserstoff entsprechen.
Eine weitere Ausführungsform betrifft Verbindungen der Formeln I und II, in denen R5 und R6 beide nicht durch Wasserstoff beschrieben werden.
Eine weitere Ausführungsform betrifft Verbindungen der Formeln I und II, in denen R2 und R3 unabhängig voneinander durch Wasserstoff, Methyl, Ethyl, Fluor, Chlor, Brom oder Trifluormethyl beschrieben werden, bevorzugt sind Verbindungen, in denen R2 und R3 unabhängig voneinander durch Wasserstoff und Methyl beschrieben werden, ganz besonders bevorzugt sind Verbindungen, in denen R2 und R3 beide durch Wasserstoff beschrieben werden.
Eine weitere Ausführungsform betrifft Verbindungen der Formeln I und II, in denen R4 durch Wasserstoff, Methyl oder Ethyl beschrieben wird, bevorzugt sind Verbindungen, in denen R4 durch Wasserstoff beschrieben wird.

Speziell bevorzugt sind Verbindungen der Formel I ausgewählt aus der Gruppe
2-(4-Methyl-3-thienylamino)-1H-thieno[3,4-d]imidazol,
2-(2,6-Dichlorphenylamino)-1H-thieno[3,4-d]imidazol,
2-(2-Chlor-4-methyl-3-thienylamino)-1H-thieno[3,4-d]imidazol,
2-(2 -Trifluormethylphenylamino)-1H-thieno[3,4-d]imidazol,
2-(2,6 -Dimethylphenylamino)-1H-thieno[3,4-d]imidazol,
2-(2,6 -Difluorphenylamino)-1H-thieno[3,4-d]imidazol,
2-(2-Chlor-6-methylphenylamino)-1H-thieno[3,4-d]imidazol,
2-(2,4-Dichlor-3-thienylamino)-1H-thieno[3,4-d]imidazol
und
2-(2-Chlor-4-methyl-3-thienylamino)-1 H-thieno[3,4-d]imidazol,
sowie deren pharmazeutisch verträgliche Salze und deren Trifluoressigsäuresalze.

Besonders speziell bevorzugt sind Verbindungen der Formel I ausgewählt aus der Gruppe
2-(2,6-Dichlorphenylamino)-1 H-thieno[3,4-d]imidazol,
2-(2-Chlor-4-methyl-3-thienylamino)-1 H-thieno[3,4-d]imidazol
und
2-(2,4-Dichlor-3-thienylamino)-1 H-thieno[3,4-d]imidazol,
sowie deren pharmazeutisch verträgliche Salze und deren Trifluoressigsäuresalze.

Enthalten die Verbindungen der Formel I oder II ein oder mehrere Asymmetriezentren, so können diese unabhängig voneinander sowohl S als auch R konfiguriert sein. Die Verbindungen können als optische Isomere, als Diastereomere, als Racemate oder als Gemische in allen Verhältnissen derselben vorliegen.

Die vorliegende Erfindung umfasst alle tautomeren Formen der Verbindungen der Formeln I und II.

Verbindungen der Formeln I und II können Säure unter Bildung von Salzen binden. Als Säureadditionssalze kommen insbesondere Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate, Adipinate, Fumarate, Gluconate, Glutamate, Glycerolphosphate, Benzoate, Oxalate, Maleinate und Pamoate, aber auch Trifluoracetate.

Alkylreste können geradkettig oder verzweigt sein. Dies gilt auch, wenn sie Substituenten tragen oder als Substituenten anderer Reste auftreten, beispielsweise in Alkoxyresten, Alkylaminoresten und Alkylsulfonylresten. Beispiele für Alkylreste sind Methyl, Ethyl, n-Propyl oder Isopropyl (= 1-Methylethyl). Bevorzugte Alkylreste sind Methyl oder Ethyl, besonders bevorzugt Methyl. In Alkylresten können ein oder mehrere, zum Beispiel 1, 2 oder 3, Wasserstoffatome durch Fluoratome substituiert sein. Beispiele für solche Fluoralkylreste sind Trifluormethyl und 2,2,2-Trifluorethyl. Substituierte Alkylreste können in beliebigen Positionen substituiert sein.

Beispiele für Cycloalkylreste sind Cyclopropyl, Cyclobutyl oder Cyclopentyl. Substituierte Cycloalkylreste können in beliebigen Positionen substituiert sein. Cycloalkylreste können auch als Substituenten anderer Reste auftreten, zum Beispiel als Cyclopropylmethyl.

Gegenstand der Erfindung ist auch das im folgenden beschriebenen Verfahren zur Herstellung der Verbindungen der Formeln I und/oder II.

Die vorliegende Erfindung betrifft ein Verfahren zur Herstellung einer Verbindung der Formeln I oder II und/oder deren pharmazeutisch verträgliche Salze, dadurch gekennzeichnet, dass man
a) ein Diamin der Formeln 111 oder IV mit einem Cyanat der Formel V zu einem Harnstoff-Derivat der Formel VI oder VII umsetzt
b) das Harnstoff-Derivat der Formel VI oder VII zu einer Verbindung der Formeln la oder IIa cyclisiert
   und
c) zur Herstellung einer Verbindung der Formeln 1 oder 11, in der R4 verschieden von Wasserstoff ist, eine Verbindung der Formeln la oder lla zu einer Verbindung der Formeln 1 oder 11 derivatisiert, 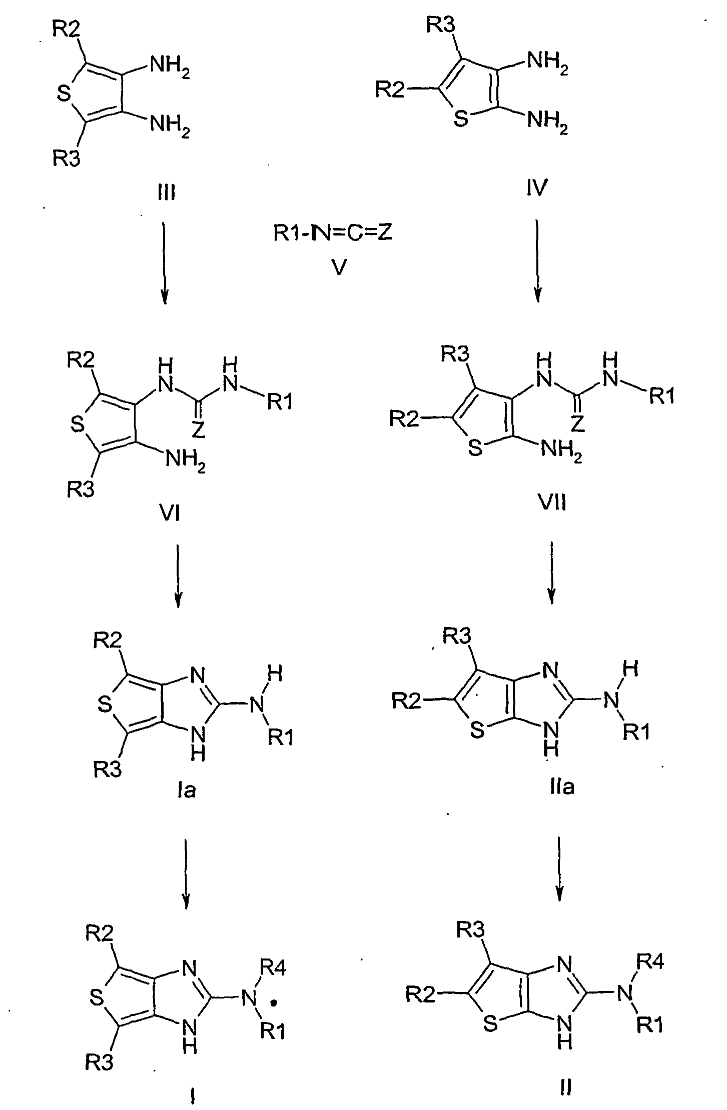
worin R1, R2, R3 und R4 die oben angegebene Bedeutung besitzen und wobei Z Sauerstoff oder bevorzugt Schwefel ist.

So lassen sich die durch Formel I oder II beschriebenen Substanzen aus Isothiocyanaten der Formel Va

R1-N=C=S Va

und den entsprechenden Diaminen der Formeln III oder IV herstellen. Zunächst wird hierfür das Diamin der Formel III oder IV in einem inerten Lösungsmittel, zum Beispiel einem Ether wie Tetrahydrofuran, mit dem entsprechenden lsothiocyanat der Formel Va umgesetzt, wobei im allgemeinen bei Temperaturen von 0°C bis 100°C, zum Beispiel bei Raumtemperatur, gearbeitet wird. Die lsothiocyanate der Formel Va können, falls nicht käuflich erhältlich, aus den entsprechenden Anilinen durch dem Fachmann bekannte Methoden, z. B. durch Behandeln mit Thiophosgen (J. Med. Chem., 1975, 18, 90-99) oder Thiocarbonyldiimidazol (Justus Liebigs Ann. Chem., 1962, 657, 104) hergestellt werden. Das hierbei intermediär gebildete Thioharnstoff-Derivat kann in einem geeigneten Lösungsmittel, zum Beispiel einem Alkohol wie Ethanol, zum Beispiel mittels Methyliodid (Synthesis, 1974, 41 - 42) oder Carbodiimid (Synthesis, 1977, 864 - 865) bei Temperaturen von 0°C bis 100°C, zum Beispiel bei der Siedetemperatur des Lösungsmittels oder bei Raumtemperatur, zur entsprechenden Verbindung der Formel la oder lla cyclisiert werden. Eine weitere Cyclisierungsmethode besteht in der Einwirkung eines Sulfonsäurechlorides, zum Beispiel Toluolsulfonsäurechlorid, auf das gebildete Thioharnstoff-Derivat in einem inerten Lösungsmittel, zum Beispiel einem Ether wie Tetrahydrofuran, bei Temperaturen zwischen 0°C und 100°C, zum Beispiel bei Raumtemperatur, wobei bevorzugt in Gegenwart einer Base, wie zum Beispiel NaOH oder KOH, gearbeitet wird.

Neben den oben beschriebenen Isothiocyanaten der Formel Va lassen sich auch die Isocyanate der Formel Vb

R1-N=C=O Vb

mit Diaminen vom Typ der Formel II oder III zu Verbindungen der Formel la oder IIa umsetzen. Dabei wird das intermediär gebildete Harnstoffderivat bevorzugt mit Phosphoroxychlorid zu den entsprechenden Verbindungen der Formel Ia oder IIa cyclisiert.

Die entsprechenden Isocyanate der Formel Vb und die Diamine der Formeln III oder IV sind käuflich erhältlich oder können nach oder analog zu in der Literatur beschriebenen, dem Fachmann bekannten Verfahren hergestellt werden.
Zur optionalen Derivatisierung werden die Verbindungen der allgemeinen Formeln la oder IIa zunächst mit einer anorganischen Base, wie zum Beispiel NaH, oder einer organischen Base bei einer Temperatur zwischen -30°C und 100°C, bevorzugt bei RT, deprotoniert und dann mit einem geeigneten Etektrophil, wie zum Beispiel Halogeniden wie Methyliodid, bei einer Temperatur zwischen -30°C und 100°C, bevorzugt bei RT, zu Derivaten der Formeln I oder II umgesetzt, in denen R4 nicht Wasserstoff ist.

Die Aufarbeitung und gewünschtenfalls die Reinigung der Produkte und/oder Zwischenprodukte erfolgt nach den üblichen Methoden wie Extraktion, Chromatographie oder Kristallisation und den üblichen Trocknungen.

Bei der vorliegenden Erfindung konnte überraschend gezeigt werden, dass die Verbindungen der Formel I und II potente Inhibitoren des Natrium-/ Protonen-Austauschers (NHE), insbesondere des Natrium-/ Protonen-Austauschers vom Subtyp 3 (NHE3), darstellen.

Die bisher bekannten NHE3-Inhibitoren leiten sich beispielsweise von Verbindungen des Acylguanidin-Typs (EP825178), Norbornylamin-Typs (WO0144164), 2-Guanidinochinazolin-Typs (WO0179186), Benzamidin-Typs (W00121582, WO0172742) oder Tetrahydroisochinolin-Typ (WO03048129, WO03055880) ab. Das ebenfalls als NHE3-Inhibitor beschriebene Squalamin (M. Donowitz et al. Am. J. Physiol. 276 (Cell Physiol. 45): C136 - C144) wirkt nach derzeitigem Wissensstand nicht unmittelbar wie die Verbindungen nach Formel I oder II, sondern über einen indirekten Mechanismus und erreicht so seine maximale Wirkstärke erst nach einer Stunde. Solche mechanistisch andersartig wirkende NHE3-Inhibitoren eignen sich beispielsweise als Kombinationspartner der vorliegenden erfindungsgemäßen Verbindungen.

Das den erfindungsgemäßen Verbindungen entfernt ähnliche Clonidin ist als schwacher NHE-Inhibitor bekannt. Allerdings ist seine Wirkung auf den NHE3 der Ratte mit einer halbmaximalen Hemmkonzentration (IC₅₀) von 620 µM äußerst moderat. Stattdessen weist es eine gewisse Selektivität für den NHE2 auf (J. Orlowski et al J. Biol. Chem. 268, 25536). Es ist daher eher als NHE2-lnhibitor zu bezeichnen. Neben der schwachen NHE-Wirkung besitzt das Clonidin eine hohe Affinität zum adrenergen alpha2-Rezeptor und Imidazolin 11-Rezeptor, wodurch eine starke blutdrucksenkende Wirkung verursacht wird (Ernsberger et al Eur. J. Pharmacol. 134, 1,1987).

Dem Clonidin ähnliche Verbindungen mit einem Thiophen- statt dem Phenylring sind aus der DE1941761 bekannt. Die hier beschriebenen Strukturen der Formel 1 oder 11 unterscheiden sich von bekannten Verbindungen durch die Annelierung eines Thieno-Restes am Imidazolteil der Formel 1 oder 11. Durch diesen Unterschied können die oben beschriebenen Clonidin-artigen, unerwünschten durch alpha-Adrenoceptorwirkung vermittelten Herz-Kreislaufeffekte eliminiert werden. Gleichzeitig werden infolge dieser Substitutionsunterschiede die NHE-inhibierenden Eigenschaften der hier beschriebenen Verbindungen bis in den mikromolaren und submikromolaren Bereich verstärkt, während die aus DE1941761 bekannten Verbindungen keine oder nur sehr schwach ausgeprägte NHE-inhibierende Effekte zeigen. So besitzt der in der Anmeldung DE1941761 beschriebenen Blutdrucksenker Tiamenidin in einem therapeutisch verwertbaren Konzentrationsbereich keine relevanten inhibitorischen Wirkungen auf die untersuchten NHE-Subtypen NHE1, NHE2, NHE3 und NHE5.

In der Anmeldung WO03053434 werden NHE3-Inhibitoren vom Imidazolidin-Typ vorgeschlagen, in der Patentanmeldung WO03101984 vom Thiophen-Typ und in der Anmeldung DE10304374 vom Imidazol-Typ. NHE3-Inhibitoren vom Benzimidazol-Typ werden in WO0246169 und DE10304294 beschrieben. Überraschend wurde nun gefunden, dass die hier beschriebenen Verbindungen der Formel I und II ebenfalls potente Inhibitoren des NHE3 darstellen und dabei vorteilhafte pharmakologische und pharmakokinetische Eigenschaften besitzen.

Der NHE3 wird im Körper verschiedener Spezies bevorzugt in der Galle, dem Darm und in der Niere gefunden (Larry Fliegel et al, Biochem. Cell. Biol. 76: 735 - 741, 1998), ließ sich aber auch im Gehirn nachweisen (E. Ma et al. Neuroscience 79: 591 - 603).

Aufgrund der NHE-inhibitorischen Eigenschaften eignen sich die Verbindungen der Formel I und II und deren pharmazeutisch verträgliche Salze zur Prävention und Behandlung von Krankheiten, die durch eine Aktivierung bzw. durch einen aktivierten NHE verursacht werden, sowie von Krankheiten, die sekundär durch die NHEbedingten Schädigungen verursacht werden.

Da NHE Inhibitoren in überwiegender Weise über die Beeinflussung der zellulären pH-Regulation wirken, können diese in günstiger Weise mit anderen, den intrazellulären pH-Wert ebenfalls regulierenden Verbindungen kombiniert werden, wobei Inhibitoren der Enzymgruppe der Carboanhydratasen, Inhibitoren der Bicarbonationen transportierenden Systeme wie des Natrium-Bicarbonat-Cotransporters (NBC) oder des Natrium abhängigen Chlorid-Bicarbonat-Austauschers (NCBE), sowie mit NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt oder moduliert werden können.

Die Verwendung der erfindungsgemäßen Verbindungen betrifft die Prävention und die Behandlung akuter und chronischer Krankheiten in der Veterinär- und in der Humanmedizin.

Die pharmakologische Wirkung der Verbindungen der Formel I oder II ist dadurch gekennzeichnet, dass sie zu einer Verbesserung des Atemantriebes führen. Sie können deshalb zur Behandlung von gestörten Atmungszuständen herangezogen werden, wie sie beispielsweise bei folgenden klinischen Zuständen und Krankheiten auftreten können: Gestörter zentraler Atemantrieb (z. B. zentrale Schlafapnoen, plötzlicher Kindstod, postoperative Hypoxie), muskulär-bedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie. Zusätzlich erhöhen die Verbindungen den Muskeltonus der oberen Atemwege, so dass das Schnarchen unterdrückt wird. Die genannten Verbindungen finden deshalb vorteilhaft Verwendung zur Herstellung eines Medikaments zur Prävention und Behandlung von Schlafapnoen und muskulär bedingter Atemstörungen und zur Herstellung eines Medikaments zur Prävention und Behandlung des Schnarchens.

Eine Kombination eines NHE-Inhibitors der Formel I oder II mit einem Carboanhydrase-Hemmer (z. B. Acetazolamid) kann sich als vorteilhaft erweisen, wobei letzterer eine metabolische Azidose herbeiführt und dadurch bereits die Atmungstätigkeit steigert, so daß eine verstärkte Wirkung und verminderter Wirkstoffeinsatz erzielt werden können.

Die erfindungsgemäßen Verbindungen schonen infolge ihrer NHE3-inhibitorischen Wirkung die zellulären Energiereserven, die sich unter toxischen und pathogenen Ereignissen rasch erschöpfen und so zur Zellschädigung oder zum Zelluntergang führen. Dabei wird die energieaufwendige ATP-verbrauchende Natriumresorption im proximalen Tubulus unter dem Einfluß von NHE3-Inhibitoren vorübergehend still gelegt und die Zelle kann so eine akute pathogene, ischämische oder toxische Situation überleben. Die Verbindungen eignen sich deshalb beispielsweise als Arzneimittel zur Behandlung ischämischer Noxen, zum Beispiel des akuten Nierenversagens. Weiterhin eignen sich die Verbindungen auch zur Behandlung aller chronischen Nierenerkrankungen und Nephritisformen, die infolge vermehrter Proteinausscheidung zum chronischen Nierenversagen führen. Dementsprechend eignen sich die Verbindungen der Formel I oder II zur Herstellung eines Medikaments zur Behandlung diabetischer Spätschäden, der diabetischen Nephropathie und chronischer Nierenerkrankungen, insbesondere von allen Nierenentzündungen (Nephritiden), die mit einer vermehrten Protein-/ Albuminausscheidung verbunden sind.

Es hat sich gezeigt, dass die erfindungsgemäß verwendeten Verbindungen eine milde abführende Wirkung besitzen und demzufolge auch vorteilhaft als Abführmittel oder bei drohender Darmverstopfung verwendet werden können.

Weiterhin können die erfindungsgemäßen Verbindungen vorteilhaft zur Prävention und Therapie von akuten und chronischen Erkrankungen des Darmtrakts verwendet werden, die beispielweise durch ischämische Zustände im Intestinalbereich und / oder durch nachfolgende Reperfusion oder durch entzündliche Zustände und Ereignisse ausgelöst werden. Solche Komplikationen können beispielweise durch mangelnde Darmperistaltik, wie sie z.B. häufig nach chirurgischen Eingriffen, bei Darmverstopfung oder stark verminderter Darmtätigkeit zu beobachten sind.

Mit den erfindungsgemäßen Verbindungen besteht die Möglichkeit, der Gallenstein-Bildung vorzubeugen.

Die erfindungsgemäßen NHE-Inhibitoren eignen sich generell zur Behandlung von Krankheiten, die durch Ischämie und durch Reperfusion hervorgerufen werden.

Die erfindungsgemäßen Verbindungen sind infolge ihrer pharmakologischen Eigenschaften als antiarrhythmische Arzneimittel geeignet. Durch ihre cardioprotektive Komponente eignen sich die NHE-Inhibitoren hervorragend zur Infarktprophylaxe und der Infarktbehandlung sowie zur Behandlung der angina pectoris, wobei sie auch präventiv die pathophys iologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I oder II infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die erfindungsgemäßen Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den mit Verbindungen der Formel I oder II vorbehandelten Empfängerorganismus verwendet werden können.

Die Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Da NHE-Inhibitoren menschliches Gewebe und Organe nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen, Anwendung finden, ist die kombinierte Verabreichung mit Verbindungen der Formel 1 oder 11 geeignet, die cytotoxischen Effekte einer Therapie zu vermindern oder zu unterdrücken. Durch die Verminderung der cytotoxischen Effekte, insbesondere der Cardiotoxizität, infolge Ko-Medikation mit NHE-Inhibitoren kann außerdem die Dosis der cytotoxischen Therapeutika erhöht und / oder die Medikation mit solchen Arzneimitteln verlängert werden. Der therapeutische Nutzen einer solchen cytotoxischen Therapie kann durch die Kombination mit NHE-Inhibitoren erheblich gesteigert werden. Die Verbindungen der Formel I oder II eignen sich insbesondere zur Verbesserung der Therapie mit Arzneimitteln, die eine unerwünschte cardiotoxische Komponente besitzen.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind.

Die Verbindungen der Formel I oder II eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychische Depressionszustände, Angsterkrankungen und Psychosen. Dabei können die erfindungsgemäßen NHE-Inhibitoren allein angewandt werden oder in Kombination mit anderen antiepileptisch wirkenden Substanzen oder antipsychotischen Wirkstoffen, oder Carboanhydratasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäßen Verbindungen der Formel I oder II ebenfalls zur Behandlung von Formen des Schocks, wie beispielweise des allergischen, cardiogenen, hypovolämischen und des bakteriellen Schocks.

Die Verbindungen der Formel 1 oder 11 können ebenfalls zur Prävention und zur Behandlung thrombotischer Erkrankungen verwendet werden, da sie als NHE-Inhibitoren sowohl die Plättchenaggregation selbst inhibieren können. Darüberhinaus können sie die nach Ischämie und Reperfusion stattfindende überschießende Freisetzung von Entzündungs- und Gerinnungsmediatoren, insbesondere des von Willebrand Faktors und thrombogener Selectin-Proteine hemmen bzw. verhindern. Damit kann die pathogene Wirkung thrombogener und entzündungsrelevanter Faktoren vermindert und ausgeschaltet werden. .Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan-Rezeptor-Antagonisten, Phosphodiesterase-Inhibitoren, Factor-Vlla-Antagonisten, Clopidogrel, Ticlopidin usw. Eine kombinierte Anwendung der vorliegenden NHE-Inhibitoren mit NCBE-Inhibitoren und/oder mit Inhibitoren der Carboanhydratase, wie beispielsweise mit Acetazolamid, ist besonders günstig.

Darüber hinaus zeichnen sich die erfindungsgemäßen NHE-Inhibitoren durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I oder II als wertvolle Therapeutika für Krankheiten in Frage, bei denen die Zellproliferation eine primäre oder sekundäre Ursache darstellt, und können deshalb als Antiatherosklerotika, Mittel gegen das chronische Nierenversagen, Krebserkrankungen, verwendet werden. Sie können somit zur Behandlung von Organhypertrophien und -hyperplasien, beispielsweise des Herzens und der Prostata verwendet werden. Verbindungen der Formel I oder II sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

NHE-Inhibitoren zeichnen sich weiterhin durch eine Verzögerung oder Verhinderung von fibrotischen Erkrankungen aus. Sie eignen sich somit als ausgezeichnete Mittel zur Behandlung von Fibrosen des Herzens, sowie der Lungenfibrose, Leberfibrose, der Nierenfibrose und anderer fibrotischer Erkrankungen.

Da der NHE bei essentiellen Hypertonikern signifikant erhöht ist, eignen sich die Verbindungen der Formel 1 oder 11 zur Prävention und zu r Behandlung des Bluthochdrucks und von Herz-Kreislauferkrankungen. Dabei können sie allein oder mit einem geeigneten Kombinations- und Formulierungspartner zur Bluthochdruckbehandlung und von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amiloride, Triamteren, Spironolacton oder Epleron, mit Verbindungen der Formel I oder II kombiniert werden. Weiterhin können die NHE-Inhibitoren der vorliegenden Erfindung in Kombination mit Calcium-Antagonisten, wie Verapamil, Diltiazem, Amlodipin, oder Nifedipin, sowie mit ACE-Hemmern, wie beispielsweise Ramipril, Enalapril, Lisinopril, Fosinopril oder Captopril verwendet werden. Weitere günstige Kombinationspartner sind auch β-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin-Rezeptoragonisten, Inhibitoren und Aktivatoren von Kaliumkanälen wie des Glibenclamid, Glimepirid, Diazoxid, Cromokalim, Minoxidil und deren Derivate, Aktivatoren des mitochondrialen ATPsensitiven Kaliumkanals (mitoK(ATP) Kanal), Inhibitoren weiterer Kaliumkanäle, wie des Kv1.5 usw.

Infolge ihrer antiphlogistischen Wirkung können erfindungsgemäße NHE-Inhibitoren als Antiinflammatorika verwendet werden. Mechanistisch fällt dabei die Inhibition der Freisetzung von Entzündungsmediatoren auf. Die Verbindungen können somit allein oder in Kombination mit einem Antiphlogistikum bei der Prävention oder Behandlung chronischer und akuter inflammatorisch er Erkrankungen verwendet werden. Als Kombinationspartner werden vorteilhaft steroidale und nicht-steroidale Antiinflammatorika verwendet.

Es wurde außerdem gefunden, dass NHE-Inhibitoren eine günstige Beeinflussung der Serumlipoproteine zeigen. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen NHE-Inhibitoren zu einer deutlichen Reduktion der durch Stoffwechselanomalien induzierten Infarkte und insbesondere zu einer signifikanten Verminderung der induzierten Infarktgröße und dessen Schweregrades. NHE-Inhibitoren der Formel I oder II finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten; die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und zur Behandlung kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I oder II mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I oder II steigert, stellt eine günstige Kombination mit verstärkter Wirkung und vermindertem Wirkstoffeinsatz dar.

So führen NHE-Inhibitoren zu einen wirksamen Schutz gegen Endothelschädigungen unterschiedlicher Genese. Mit diesem Schutz der Gefäße gegen das Syndrom der endothelialen Dysfunktion sind NHE-Inhibitoren wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem eignen sich NHE-Inhibitoren zur Behandlung des nicht-insulinabhängigen Diabetes (NIDDM), wobei beispielweise die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburid, Glimepirid, Tolbutamid usw., einem.Glucosidase Inhibitor, einem PPAR-Agonisten, wie Rosiglitazone, Pioglitazone etc, mit einem Insulinpräparat unterschiedlicher Verabreichungsform, mit einem DB4 Inhibitor, mit einem Insuli nsensitizer oder mit Meglitinide zu kombinieren.

Neben den akuten antidiabetischen Effekten wirken NHE-Inhibitoren der Entstehung diabetischer Spätkomplikation entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den oben unter NIDDM-Behandlung beschriebenen antidiabetischen Arzneimitteln vorteilhaft kombiniert werden. Der Kombination mit einer günstigen Darreichungsform von Insulin kann dabei eine besondere Bedeutung zukommen.

NHE-Inhibitoren zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die auch unabhängig von akuten Mangeldurchblutungszuständen sind und auch bei normalen, nicht-ischämischen Bedingungen auftreten können. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen.
Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. NHE-Inhibitoren eignen sich somit hervorragend zur Behandlung und Prävention der altersbedingt fortschreitenden endothelialen Dysfunktion, insbesondere von claudicatio intermittens. Außerdem eignen sich NHE-Inhibitoren somit hervorragend zur Behandlung und Prävention der Herzinsuffizienz, des congestive heart failure (CHF) sowie zur Behandlung und insbesondere zur Prävention von altersbedingten Formen von Krebs.
Dabei kommt eine Kombination mit blutdrucksenkenden Medikamenten, wie mit ACE-Hemmern, Angiotensinrezeptorantagonisten, Diuretika, Ca⁺²-Antagonisten etc. oder mit stoffiwechselnormalisierenden Medikamenten, wie Cholesterinsenkern, in Betracht. Die Verbindungen der Formel 1 oder 11 eignen sich somit zur Prävention altersbedingter Gewebsveränderungen und zur Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Weiterhin sind NHE3-Inhibitoren zur Behandlung von durch Bakterien sowie durch Protozoen ausgelösten Erkrankungen (human und veterinär) geeignet. Bei den durch Protozoen ausgelösten Erkrankungen sind insbesondere Malariaerkrankungen des Menschen und Hühnercoccidiose zu nennen.
Außerdem eignen sich die Verbindungen als Mittel zur Bekämpfung von saugenden Parasiten in der Human- und Veterinärmedizin sowie im Pflanzenschutz. Dabei ist die Verwendung als Mittel gegen blutsaugende Parasiten in der Human- und Veterinärmedizin bevorzugt.

Beansprucht wird weiterhin ein Heilmittel für die humane, veterinäre oder phytoprotektive Anwendung, enthaltend eine wirksame Menge einer Verbindung der Formel I und II und/oder deren pharmazeutisch verträgliche Salze, zusammen mit pharmazeutisch annehmbaren Träger- und Zusatzstoffen, allein oder in Kombination mit anderen pharmakologischen Wirkstoffen oder Arzneimitteln.

Arzneimittel, die eine Verbindung der Formel I oder II oder deren pharmazeutisch verträgliche Salze enthalten, können zum Beispiel oral, parenteral, intramuskulär, intravenös, rektal, nasal, durch Inhalation, subkutan oder durch eine geeignete transkutane Darreichungsform appliziert werden, wobei die bevorzugte Applikation von dem jeweiligen Erscheinungsbild der Erkrankung abhängig ist. Die Verbindungen der Formel I oder II können dabei allein oder zusammen mit galenischen Hilfsstoffen zur Anwendung kommen, und zwar sowohl in der Veterinär- als auch in der Humanmedizin und im Pflanzenschutz. Die Arzneimittel enthalten Wirkstoffe der Formel I und II und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln , wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, perkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I oder II in einem pharmazeutisch unbedenklichen Lösungsmittels, wie insbesondere Ethanol oder Wasser, oder einem Gemisch solcher Lösungsmittel.
Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I oder II und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I oder II bei einem etwa 75 kg schweren Patienten mindestens 0,001 mg/kg, vorzugsweise 0,1 mg/kg, bis höchstens 30 mg/kg, vorzugsweise 1 mg/kg Körpergewicht. In akuten Situationen, etwa unmittelbar nach Erleiden apnoetischer Zustände im Hochgebirge, können auch noch höhere Dosen notwendig werden. Insbesondere bei i.v.
Anwendung, etwa bei einem Infarktpatienten auf der Intensivstation können bis zu 300 mg/kg pro Tag notwendig werden. Die Tagesdosis kann auf eine oder mehrere, zum Beispiel bis zu 4 Einzeldosen verteilt werden.

Versuchsbeschreibungen und Beispiele:

### Beispiel 1: 2-(4-Methyl-3-thienylamino)-1 H-thieno[3,4-d]imidazol Hydrochlorid

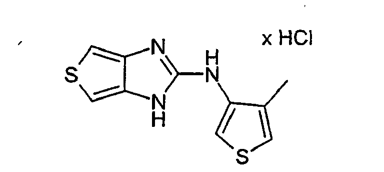

### a) N-(3-Amino-4-thienyl)-N'-(4-methyl-3-thienyl)-thioharnstoff

Eine Mischung aus 1,87 g 3,4-Diaminothiophen Dihydrochlorid, 60ml wasserfreiem Tetrahydrofuran (THF) und 2,58g N-Ethyl-N,N-diisopropyl-amin wurde 30 Minuten bei Raumtemperatur gerührt und sodann mit 1,5 g 4-Methyl-3-thienylisothiocyanat versetzt. Nach Rührung über ca. 18 Stunden wurde das Lösungsmittel abdestilliert, der Rückstand mit Wasser versetzt und mehrfach mit Ethylacetat extrahiert. Nach dem Behandeln der vereinigten organischen Phasen mit Aktiv-Kohle trocknete man über Natriumsulfat und destillierte erneut das Lösungsmittel ab. Der N-(3-Amino-4-thienyl)-N'-(4-methyl-3-thienyl)-thioharnstoff wurde als amorpher schaumiger Feststoff erhalten.
Zur weiteren Charakterisierung wurden 0,15 g N-(3-Amino-4-thienyl)-N'-(4-methyl-3-thienyl)-thioharnstoff in 20 ml Ethylacetat mit einer Chlorwasserstoffgas-gesättigten Etherlösung stark sauer gestellt und der Feststoff abfiltriert.
Hygroskopisches Produkt, Zersetzungspunkt ab 110°C.

### b) 2-(4-Methyl-3-thienylamino)-1 H-thieno[3,4-d]imidazol Hydrochlorid

Zu einer Suspension aus 1,2 g N-(3-Amino-4-thienyl)-N'-(4-methyl-3-thienyl)-thioharnstoff in 40 ml wasserfreiem Ethanol gab man 5,05 g Methyliodid und kochte 5 Stunden unter Rückfluß. Nach Abdestillieren des Lösungsmittels am Rotavapor versetzte man den Rückstand mit Wasser, stellte mit einer wäßrigen gesättigten Natriumhydrogencarbonat-Lösung alkalisch und extrahierte mehrmals mit Ethylacetat. Die vereinigten organischen Phasen wurden über Natriumsulfat getrocknet, mit Aktivkohle behandelt und das Lösungsmittel am Rotavapor abdestilliert. Der Rückstand wurde sodann durch Säulenchromatografie an Kieselgel mit einem Gemisch aus 10 Volumenteilen Dichlormethan und 1 Volumenteil Methanol gereinigt und nach erneutem Abdestillieren des Lösungsmittels unter Diisopropylether zur Kristallisation gebracht. Brauner Feststoff, Zersetzungspunkt 105°C.

### Beispiel 2: 2-(2,6-Dichlorphenylamino)-H-thieno[3,4-d]imidazol Hydrochlorid

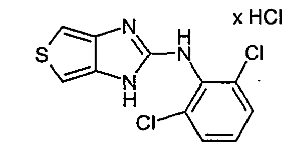

### a) N-(3-Amino-4-thienyl)-N'-(2,6-dichlorphenyl)-thioharnstoff

Eine Mischung aus 1,87 g 3,4-Diaminothiophen Dihydrochlorid, 60ml wasserfreiem THF und 2,58g N-Ethyl-N,N-diisopropyl-amin wurde 30 Minuten bei Raumtemperatur gerührt und sodann mit 2,04 g 2,6-Dichlorphenylisothiocyanat versetzt. Nach Erwärmen für 10 Mi nuten auf 40°C rührte man ca. 18 Stunden bei Raumtemperatur, destilliert das Lösungsmittel ab, versetzte den Rückstand mit Wasser und extrahierte mehrfach mit Ethylacetat. Nach dem Behandeln der vereinigten organischen Phasen mit Aktiv-Kohle trocknete man über Natriumsulfat und destilliert erneut das Lösungsmittel ab. Man erhielt einen kristallinen Feststoff, den man nach Verrühren mit wenig Ethylacetat abfiltrierte. Schmp. 165°C.

### b) 2-(2,6-Dichlorphenylamino)-1H-thieno[3,4-d]imidazol Hydrochlorid

Zu einer Suspension aus 0,951g N-(3-Amino-4-thienyl)-N'-(2,6-dichlorphenyl)-thioharnstoff in 40ml wasserfreiem Ethanol gab man 2,5 g Methyliodid und kochte 6 Stunden am Rückflußkühler. Nach Abdestillieren des Lösungsmittels am Rotavapor versetzte man den Rückstand mit Wasser, stellte mit einer wäßrigen gesättigten Natriumhydrogencarbonat-Lösung alkalisch und extrahierte mehrmals mit Ethylacetat. Die vereinigten organischen Phasen wurden über Natriumsulfat getrocknet, mit Aktivkohle behandelt und das Lösungsmittel am Rotavapor abdestilliert. Der Rückstand wurde sodann durch Säulenchromatografie an Kieselgel mit einem Gemisch aus 10 Volumenteilen Dichlormethan und 1 Volumenteil Methanol gereinigt. Nach Abdestillieren des Lösungsmittels erhielt man ein braunes ölig-amorphes Produkt. Man löste in wenig Ethylacetat, stellte mit einer Chlorwasserstoffgas-gesättigten Etherlösung stark sauer, und kochte nach Zugabe von wenig Aceton bis zur vollständigen Kristallisation. Schmp. > 300°C.

### Beispiel 3: 2-(2,6-Dichlorphenylamino)-4,6-dimethyl-1 H-thieno[3,4-d]imidazol Hydrochlorid

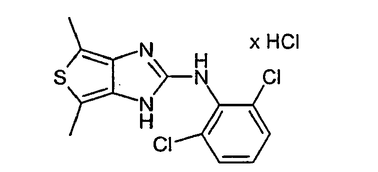

### a) N-(3-Amino-2,5-dimethyl-4-thienyl)-N'-(2,6-dichlorphenyl)-thioharnstoff

Eine Mischung aus 0,5 g 3,4-Diamino-2,5-dimethylthiophen Dihydrochlorid, 30ml wasserfreiem THF und 0,49 g Triethylamin wurde 30 Minuten bei Raumtemperatur gerührt und sodann mit 0,569 g 2,6-Dichlorphenylisothiocyanat versetzt. Nach Erwärmen für 30 Minuten auf 40°C rührte man ca. 18 Stunden bei Raumtemperatur, destillierte das Lösungsmittel ab, versetzte den Rückstand mit Wasser und filtrierte den Niederschlag ab, den man durch Säulenchromatografie an Kieselgel mit einem Gemisch, bestehend aus gleichen Volumenteilen Toluol und Ethylacetat, reinigte. Nach dem Abdestillieren des Lösungsmittels erhielt man ein schaumig amorphes Produkt vom Schmp. 143 - 148°C, das sich nach Schmelzen erneut verfestigte mit einem neuen Schmp. >300°C.

### b) 2-(2,6-Dichlorphenylamino)-4,6-dimethyl-1H-thieno[3,4-d]imidazol Hydrochlorid

Zu einer Lösung aus 0,27 g N-(3-Amino-2,5-dimethyl-4-thienyl)-N'-(2,6-dichlorphenyl)-thioharnstoff in 30ml wasserfreiem Ethanol gab man 0,664 g Methyliodid und kochte ca 10 Stunden unter Rückflußbedingungen. Nach Abdestillieren des Lösungsmittels am Rotavapor versetzte man den Rückstand mit Wasser und extrahierte mehrmals mit Ethylacetat. Die vereinigten organischen Phasen wurden über Natriumsulfat getrocknet und das Lösungsmittel am Rotavapor abdestilliert. Der Rückstand wurde sodann durch Säulenchromatografie an Kieselgel mit einem Gemisch aus 20 Volumenteilen Ethylacetat, 10 Volumenteilen n-Heptan, 3 Volumenteilen Eisessig gereinigt. Nach Abdestillieren des Lösungsmittels löste man in wenig Ethylacetat, stellte mit einer Chlorwasserstoffgas-gesättigten Diethylether-Lösung stark sauer, und brachte den amorphen Nierderschlag durch Erwärmen zur vollständigen Kristallisation. Schmp. 270 - 273°C.

### Beispiel 4: 2-(4-Methyl-3-thienylamino)-4,6-dimethyl-1H-thieno[3,4-d]imidazol Hydrochlorid

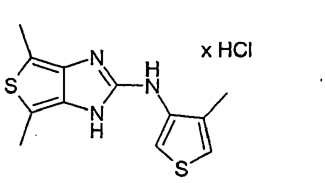

### a) N-(3-Amino-2,5-dimethyl-4-thienyl)-N'-(4-methyl-3-thienyl)-thioharnstoff

Eine Mischung aus 1 g 3,4-Diamino-2,5-dimethylthiophen Dihydrochlorid, 60ml wasserfreiem THF und 1,05 g Triethylamin wurde 30 Minuten bei Raumtemperatur gerührt und sodann mit 0,902 g 4-Methylthiophen-3-isothiocyanat versetzt. Nach Erwärmen für 15 Minuten auf 35 - 40°C rührte man ca. 18 Stunden destillierte das Lösungsmittel ab, versetzte den Rückstand mit Wasser und extrahierte mehrfach mit Ethylacetat. Nach dem Abdestillieren des Lösungsmittels der vereinigten organischen Phasen reinigte man den Rückstand durch Säulenchromatografie an Kieselgel mit einem Lösungsmittelgemisch aus gleichen Teilen Toluol und Ethylacetat. Nach Abdestillieren des Lösungsmittels unter vermindertem Druck erhielt man ein teilweise kristallisierendes Öl, das ohne weitere Reinigung weiter umgesetzt wurde.

### b) 2-(4-Methyl-3-thienylamino)-4,6-dimethyl-1 H-thieno[3,4-d]imidazol Hydrochlorid

Zu einer Suspension aus 0,34 g N-(3-Amino-2,5-dimethyl-4-thienyl)-N'-(4-methyl-3-thienyl)-thioharnstoff in 40ml wasserfreiem Ethanol gab man 0,97 g Methyliodid und kochte 6 Stunden unter Rückfluß. Nach Abdestillieren des Lösungsmittels am Rotavapor versetzte man den Rückstand mit Wasser, stellte mit einer wäßrigen gesättigten Natriumhydrogencarbonat-Lösung alkalisch und extrahierte mehrmals mit Ethylacetat. Die vereinigten organischen Phasen wurden über Natriumsulfat getrocknet, mit Aktivkohle behandelt und das Lösungsmittel am Rotavapor abdestilliert. Der Rückstand wurde sodann durch Säulenchromatografie an Kieselgel mit einem Gemisch, bestehend aus 20 Volumenteilen Ethylacetat und 10 Volumenteilen n-Heptan und 3 Volumenteilen Eisessig, gereinigt. Nach Abdestillieren des Lösungsmittels erhielt man durch Verreiben unter Diisopropylether ein braunes kristallines Produkt. Schmp. 159 - 164 °C.
Man löste in wenig Ethylacetat, stellte mit einer Chlorwasserstoffgas-gesättigten Diethylether-Lösung stark sauer, und erwärmte zur Ausfällung des bräunlich gefärbten, kristallinen Endproduktes. Schmp. 230 - 232°C.

### Beispiel 5: 2-(2-Chlor-4-methyl-3-thienylamino)-1 H-thieno[3,4-d]imidazol Hydrochlorid

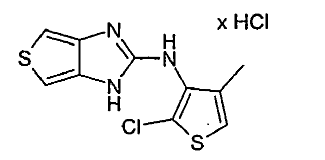

### a) 4-Methyl-3-trifluoracetylaminothiophen

Zu einer Suspension von 300mg 3-Amino-4-methylthiophen in 20ml wasserfreiem THF gab man 609 mg (= 0,83ml) Triethylamin, und nach 10 Minuten Rühren 464 mg (= 0,307ml) Trifluoressigsäureanhydrid. Nach dem Abklingen der exothermen Reaktion rührte man weitere 2 Stunden bei Raumtemperatur und ließ über Nacht stehen. Nach Abdestillieren des Lösungsmittels löste man den öligen Rückstand in Ethylacetat, wusch die organische Phase einmal mit Wasser und einmal mit verdünnter Salzsäure und nochmals mit Wasser, trocknete die organische Phase über Natriumsulfat und destillierte das Lösungsmittel ab.
Das als dunkles ölig-amorphes Produkt erhaltene 4-Methyl-3-trifluoracetylaminothiophen wurde ohne weitere Reinigung für die nächste Stufe verwendet.

### b) 2-Chlor-4-methyl-3-trifluoracetylaminothiophen

Zu einer Mischung aus 5g 4-Methyl-3-trifluoracetylaminothiophen in 70 ml Eisessig tropfte eine Lösung aus 3,2g N-Chlorsuccinimid in 50 ml Eisessig, rührte sodann 30 Minuten bei Raumtemperatur und eine weitere Stunde bei 50°C. Man destillierte das Lösungsmittel ab, versetzte den Rückstand mit Wasser und stellte mit 2N NaOH auf pH10. Man extrahierte mit Ethylacetat, wusch die organische Phase mit Wasser und trocknete über Natriumsulfat. Nach dem Abdestillieren des Lösungsmittels erhielt man ein dunkel gefärbtes öliges Produkt, das an Kieselgel mit einem Elutionsgemisch aus 10 Volumenteilen Toluol und 2 Volumenteilen n-Heptan chromatografisch gereinigt wurde. Nach dem Abdestillieren des Lösungsmittels erhielt man das Produkt als hellgelb gefärbtes Öl.

### c) 3-Amino-2-chlor-4-methylthiophen Hydrochlorid

400 mg 2-Chlor-4-methyl-3-trifluoracetylaminothiophen wurden mit 1,5 ml Hydrazinhydrat versetzt und sodann eine Stunde bei 50°C gerührt. Man versetzte mit Wasser, extrahierte die Lösung mit Ethylacetat und wusch die organische Phase einmal mit verdünnter Essigsäure und ein weiteres mal mit Wasser. Nach dem Trocknen der organischen Phase über Natriumsulfat stellte man mit Chlorwassersto-ff gesättigtem Diethylether stark sauer, und destillierte unter vermindertem Druck am Rotavapor das Lösungsmittel ab. Nach weitgehendem Abdestillieren versetzte man erneut mit Ethylacetat und destillierte erneut das Lösungsmittel ab, wobei es zur Abscheidung braun gefärbter Kristalle kam. Man filtrierte den Feststoff rasch ab und überführte zur Trocknung über Diphosphorpentoxid in einen Exsiccator. Braun gefärbter, kristalliner, hygroskopischer Feststoff. Sublimationspunkt: 120°C

### d) (2-Chlor-4-methyl-3-thienyl)-isothiocyanat

Zu einem Gemisch, dargestellt aus einer Lösung von 0,57g Natriumhydrogencarbonat in 6 ml Wasser, 20 ml Methylenchlorid und 0,34g Thiophosgen, tropfte man innerhalb von 15 Minuten eine Mischung hergestellt aus 0,5 g 3-Amino-2-chlor-4-methylthiophen Hydrochlorid, 8 ml Methylenchlorid und 0,2 ml Wasser. Man rührte 30 Minuten bei Raumtemperatur, trennte sodann die organische Phase ab und extrahierte die wäßrige Phase nochmals mit Methylenchlorid. Die vereinigten organischen Phasen wurden ein weiteres mal mit Wasser gewaschen, über Natriumsulfat getrocknet und das Lösungsmittel abdestilliert. Das so als dunkles Öl erhaltene Isothiocyanat wurde wegen seiner Empfindlichkeit ohne weitere Reinigung für die weiteren Reaktionen verwendet.

### e) N-(4-Amino-3-thienyl)-N'-(2-chlor-4-methyl-3-thienyl)-thioharnstoff

Zu einer Lösung aus 0,51g N-Ethyl-N,N-düsopropylamin in 30ml wasserfreiem THF gab man 0,55 g 3,4-Diaminothiophen Dihydrobromid, rührte ca 5 Minuten bei Raumtemperatur und versetzte die Lösung sodann mit einer Mischung aus 0,4 g (2-Chlor-4-methyl-3-thienyl)-isothiocyanat in 5 ml THF. Das Reaktionsgemisch wurde 3 Stunden bei Raumtemperatur und 10 Minuten bei 40°C gerührt. Nach dem Stehenlassen über Nacht destillierte man das Lösungsmittel ab, behandelte den Rückstand mit Wasser und extrahierte mit Ethylacetat. Man filtrierte vom unlöslichen Anteil ab, wusch die organische Phase mit Wasser und trocknete über Natriumsulfat. Nach dem Abdestillieren des Lösungsmittels reinigte man durch Säulenchromatografie an Kieselgel mit einer Mischung von 1 Volumenteil Toluol und 1 Volumenteil Ethylacetat und erhielt nach Abdestillieren des Lösungsmittels den N-(4-Amino-3-thienyl)-N'-(2-chlor-4-methylthienyl)-thioharnstoff als kristallinen Feststoff. Schmp.: 132 - 135 °C.

### f) 2-(2-Chlor-4-methyl-3-thienylamino)-1H-thieno[3,4-d]imidazol Hydrochlorid

Zu einer Mischung, dargestellt aus einer Lösung von 157 mg N-(4-Amino-3-thienyl)-N'-(2-chlor-4-methylthienyl)-thioharnstoff in 15 ml THF und einer Lösung aus 51,08 mg NaOH in 2ml Wasser, tropfte man eine Lösung aus 108,4 mg p-Toluolsulfonsäurechlorid in 5 ml wasserfreiem THF. Die erhaltene dunkle Lösung wurde 2 Stunden bei Raumtemperatur gerührt, das Lösungsmittel abdestilliert und der Rückstand mit Wasser versetzt. Nach Extraktion mit Ethylacetat wusch man die organische Phase mit Wasser und trocknete sie über Natriumsulfat. Nach dem Abdestillieren des Lösungsmittels reinigte man durch Säulenchromatografie an Kieselgel mit einem Gemisch von 20 Volumenteilen Ethylacetat, 10 Volumenteilen n-Heptan, 3 Volumenteilen Eisessig als Elutionsmittel, das sodann unter vermindertem Druck abdestilliert wurde. Man erhielt ein viskoses amorphes Produkt, das in Ethylacetat gelöst und mit Chlorwasserstoffgas gesättigtem Diethylether stark sauer gestellt wurde. Das sich abscheidende amorphe salzsaure Salz wurde durch Anreiben zur Kristallisation gebracht. Schmp.: > 300°C.

### Beispiel 6: 2-(2 -Trifluormethylphenylamino)-1H-thieno[3,4-d]imidazol Hydrochlorid

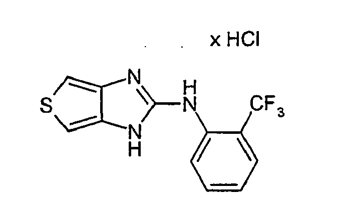

### a) N-(3-Amino-4-thienyl)-N'-(2-trifluormethylphenyl)thioharnstoff

Zu einer Mischung aus 0,74g N-Ethyl-N,N-diisopropylamin in 40ml wasserfreiem TH F gab man 0,8g 3,4-Diaminothiophen Dihydrobromid, ließ 10 Minuten bei Raumtemperatur rühren, und versetzte sodann mit 0,58g 2-Trifluormethylphenylisothiocyanat. Man ließ 3 Stunden bei Raumtemperatur, weitere 10 Minuten bei 40°C rühren und über Nacht bei Raumtemperatur stehen. Nach dem Abdestillieren des Lösungsmittels wurde der Rückstand mit Wasser versetzt, mit Essigester extrahiert und vom unlöslichen Anteil abfiltriert. Die organische Phase wurde mit Wasser gewaschen, über Natriumsulfat getrocknet, das Lösungsmittel abdestilliert und der Rückstand durch Säulenchromatografie an Kieselgel mit einem Gemisch aus 1 Volumenanteil Toluol und 1 Volumenanteil Essigester gereinigt. Kristalliner Feststoff vom Schmp. 122 - 128°C

### b) 2-(2 -Trifluormethylphenylamino)-1 H-thieno[3,4-d]imidazol Hydrochlorid

Zu einer Mischung, dargestellt aus einer Lösung von 250 mg N-(3-Amino-4-thienyl)-N'-(2-trifluormethylphenyl)thioharnstoff in 15ml THF und einer Lösung aus 77,8 mg NaOH in 3 ml Wasser, tropfte man eine Lösung von 165,3 mg p-Toluolsulfonsäurechlorid in 5 ml THF. Nach 2 Stunden Rühren bei Raumtemperatur wurde das Lösungsmittel abdestilliert, der Rückstand mit Wasser versetzt und mit Ethylacetat extrahiert. Nach dem Waschen wurde die organische Phase über Natriumsulfat getrocknet, das Lösungsmittel abdestilliert und der Rückstand durch Säulenchromatografie an Kieselgel mit einem Gemisch von 20 Volumenteilen Ethylacetat, 10 Volumenteilen n-Heptan, 3 Volumenteilen Eisessig gereinigt. Nach dem Abdestillieren des Lösungsmittels der mit LC-MS -Technik identifizierten Fraktion löste man den Rückstand in wenig Ethylacetat, stellte mit einer Lösung aus Chlorwasserstoffgas in Diethylether stark sauer und filtrierte die Kristalle nach dem Stehenlassen über Nacht ab. Kristalliner Feststoff, Schmp 194-196 °C.

### Beispiel 7: 2-(2,6 -Dimethylphenylamino)-1 H-thieno[3,4-d]imidazol Hydrochlorid

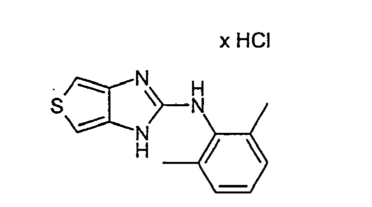

### a) N-(3-Amino-4-thienyl)-N'-(2,6-dimethylphenyl)thioharnstoff

erhielt man analog der in Beispiel 6 a) angegebenen Vorschrift durch Reaktion einer Mischung, dargestellt aus 0,74g N-Ethyl-N,N-diisopropylamin in 40ml THF, 0,8 g 3,4-Diaminothiophen Dihydrobromid und 0,47g 2,6-Dimethylphenyl-isothiocyanat. Kristalliner Feststoff. Schmp. 188 - 191°C.

### b) 2-(2,6 -Dimethylphenylamino)-1 H-thieno[3,4-d]imidazol Hydrochlorid

erhielt man analog der in Beispiel 6b angegebenen Vorschrift aus 380mg N-(3-Amino-4-thienyl)-N'-(2,6-dimethyl phenyl)thioharnstoff, 135,4 mg NaOH und 287,3 mg p-Toluolsulfonsäurechlorid. Kristalline Substanz. Schmp. >310°C.

### Beispiel 8: 2-(2,6 -Difluorphenylamino)-1 H-thieno[3,4-d]imidazol Hydrochlorid

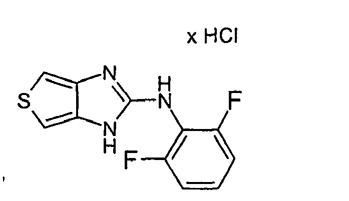

### a) N-(3-Amino-4-thienyl)-N'-(2,6-difluorphenyl)thioharnstoff

erhielt man analog der in Beispiel 6 a) angegebenen Vorschrift durch Reaktion einer Mischung, dargestellt aus 0,74 g (= 1,01ml) N-Ethyl-N,N-diisopropylamin in 40ml THF, 0,8 g 3,4-Diaminothiophen Dihydrobromid und 0,496 g 2,6-Dimethylphenyl-isothiocyanat.
Kristalliner Feststoff. Schmp. 135 - 140°C.

### b) 2-(2,6 -Difluorphenylamino)-1 H-thieno[3,4-d]imidazol Hydrochlorid

erhielt man analog der in Beispiel 6b angegebenen Vorschrift aus 335 mg N-(3-Amino-4-thienyl)-N'-(2,6-dimethylphenyl)thioharnstoff, 81,36 mg NaOH und 172,7 mg p-Toluolsulfonsäurechlorid. Kristalline Substanz. Schmp. 296°C.

### Beispiel 9: 2-(2-Chlor-6-methylphenylamino)-1H-thieno[3,4-d]imidazol Hydrochlorid

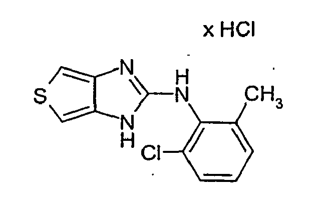

### a) N-(3-Amino-4-thienyl)-N'-(2-chlor-6-methylphenyl)thioharnstoff

erhielt man analog der in Beispiel 6 a) angegebenen Vorschrift durch Reaktion einer Mischung, dargestellt aus 0,74 g (= 1,01m1) N-Ethyl-N,N-diisopropylamin in 40ml THF, 0,8 g 3,4-Diaminothiophen Dihydrobromid und 0,473 g 2-Chlor-6-methylphenyl-5 isothiocyanat.
Kristalliner Feststoff. Schmp. 168 - 170°C.

### b) 2-(2-Chlor-6-methylphenyl)-1 H-thieno[3,4-d]imidazol Hydrochlorid

erhielt man analog der in Beispiel 6b angegebenen Vorschrift aus 170 mg N-(3-Amino-4-thienyl)-N'-(2-chlor-6-methylphenyl)thioharnstoff, 56,4 mg NaOH und 119,7 mg p-Toluolsulfonsäurechlorid. Kristalline Substanz. Schmp. >310°C.

### Beispiel 10: 2-(2,4-Dichlor-3-thienylamino)-1H-thieno[3,4-d]imidazol Hydrochlorid

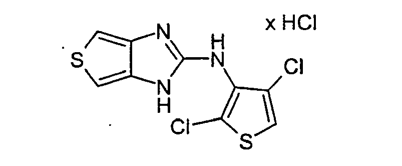

### a) 3-Acetylaminothiophen-2-carbonsäuremethylester

erhielt man durch Umsetzung von 471g 3-Aminothiophen-2-carbonsäuremethylester mit 226,64 ml Acetanhydrid in 500ml Toluol. Farbloses kristallines Produkt. Schmp. 94 - 95°C.

### b) 3-Acetylamino-4,5-dichlorthiophen-2-carbonsäuremethylester

erhielt man durch Reaktion von 19,9 g 3-Acetylaminothiophen-2-carbonsäuremethylester in 100ml Chloroform mit 17,9 ml Sulfurylchlorid (SO₂Cl₂) in 120ml Chloroform durch Rühren 2 Stunden bei 40°C und anschließendem 15 Minuten Kochen unter Rückflußbedingungen. Nach Abdestillieren des Lösungsmittels kristallisierte man in Ethylacetat. Kristalliner Feststoff. Schmp. 136 - 138 °C.

### c) 3-Acetylamino-4-chlorthiophen-2-carbonsäuremethylester

erhielt man durch katalytische Hydrierung einer Mischung aus 25 g 3-Acetylamino-4,5-dichlorthiophen-2-carbonsäuremethylester in 300ml Methanol, 9,5 g Triethylamin und mit 10g Palladium auf Kohle bei Raumtemperatur und 760 mm Hg-Säule (Normaldruck-Bedingungen). Nach Aufnahme der berechneten Menge Wasserstoff filtrierte man vom Katalysator ab, destillierte das Lösungsmittel am Rotavapor ab und brachte das Produkt unter Wasser zur Kristallisation.
Farblose kristalline Substanz. Schmp. 143-147°C.

### d) 3-Amino-4-chlorthiophen-2-carbonsäuremethylester

erhielt man durch Rühren einer Lösung von 7g 3-Acetylamino-4-chlorthiophen-2-carbonsäuremethylester in einer Mischung aus 50 ml Methanol und 50ml konzentrierter Salzsäure 4 Stunden bei 60°C und 2 Tage bei Raumtemperatur. Man filtrierte, destillierte das Lösungsmittel auf etwa 1/3 des Ausgangsvolumens ab, fügte ca 100ml Wasser zu und filtrierte die Kristalle nach dem Rühren bei Raumtemperatur ab. Kristallines Produkt. Schmp. 62 - 64 °C.

### e) 3-Amino-4-chlorthiophen

erhielt man durch Erhitzen von 19,9 g 3-Amino-4-chlorthiophen-2-carbonsäuremethylester in einer Lösung aus 16,4g Kaliumhydroxid in 150ml Wasser unter Rückflußbedingungen 90 Minuten. Man kühlte auf 60°C ab und tropfte vorsichtig unter Rühren und unter Beibehaltung der Temperatur ca 170ml wässrige 2N HCl zu dem Reaktionsgemisch, wobei infolge Decarboxylierung starkes Schäumen stattfand. Man rührte weitere 30 Minuten, kühlte ab, filtrierte den braunen Niederschlag ab, stellte das Filtrat mit NaOH auf pH 11-12 und extrahierte 3-4 mal mit Methylenchlorid. Man wusch die vereinigten organischen Phasen mit Wasser, trocknete über Natriumsulfat und erhielt nach Abdestillieren des Lösungsmittels ein braunes Öl das ohne weitere Reinigung verwendet wurde und unter Argon als Schutzgas aufbewahrt wurde.

### f) 4-Chlor-3-trifluoracetylaminothiophen

erhielt man durch Zugabe von 2,29g Trifluoracetanhydrid zu einer Lösung aus 2 g 3-Amino-4-chlorthiophen und 4,54 g Triethylamin in 40ml wasserfreiem THF. Exotherme Reaktion. Man rührte sodann 3 Stunden bei Raumtemperatur, destillierte das Lösungsmittel ab, versetzte den Rückstand mit Wasser und extrahierte mit Essigester. Nach dem Waschen der organischen Phase mit Wasser und anschließendem' Trocknen über Natriumsulfat wurde das Lösungsmittel abdestilliert. Man erhielt das gewünschte Produkt als öliges Material, das ohne weitere Reinigung weiter verarbeitet wurde.

### g) 2,4-Dichlor-3-trifluoracetylaminothiophen

Zu einer Lösung aus 2,3 g 4-Chlor-3-trifluoracetylaminothiophen in 60ml Eisessig tropfte man unter Rührung eine Lösung aus 1,4g N-Chlorsuccinimid in 25 ml Eisessig, rührte weitere 35 Minuten bei 40-45°C und destillierte das Lösungsmittel ab. Der Rückstand wurde mit Wasser versetzt und mit Ethylacetat extrahiert, die organische Phase mit Wasser gewaschen, über Natriumsulfat getrocknet und das Lösungsmittel abdestilliert. Der Rückstand wurde durch Säulenchromatografie an Kiesel gel mit einem Gemisch aus 10 Volumenteilen Toluol und 2 Volumenteilen n-Heptan gereinigt und von der noch unumgesetzten Monochlor-Verbindung abgetrennt.
Kristalline Verbindung. Schmp. 56 - 58°C.

### h) 3-Amino-2,4-dichlorthiophen

Eine Mischung aus 0,26 g 2,4-Dichlor-3-trifluoracetylaminothiophen und 2,0 ml 80%ige Hydrazinhydrat-Lösung wurde 1 Stunde bei 50°C gerührt und nach dem Stehenlassen über Nacht bei Raumtemperatur mit Wasser versetzt. Man extrahierte mit Ethylacetat, wusch mit Wasser, trocknete die organische Phase über Natriumsulfat und destillierte das Lösungsmittel ab. Kristalliner Feststoff. Schmp.: 50 - 52°C.

### i) 2,4-Dichlor-3-th ienyl-isothiocyanat

Zu einer Mischung aus 8 ml Methylenchlorid, 0,825g Natriumhydrogencarbonat gelöst in 6 ml Wasser und 0,496g Thiophosgen tropfte man im Verlauf von 15 Minuten eine Lösung aus 0,66 g 3-Amino-2,4-dichlorthiophen in 15 ml Methylenchlorid. Nach dem Rühren über 2-3 Stunden bei Raumtemperatur trennte man die organische Phase ab und extrahierte die wässrige Phase weitere zweimal mit Methylenchlorid. Die vereinigten organischen Phasen wurden mit Wasser gewaschen, über Natriumsulfat getrocknet und das Lösungsmittel abdestilliert. Man erhielt das Isothiocyanat als dunkel gefärbtes Produkt, das man infolge seiner Empfindlichkeit ohne Reinigungsoperationen weiter umsetzte.

### j) N-(4-Amino-3-thienyl)-N'-(2,4-dichlor-3-thienyl)thioharnstoff

erhielt man analog der in Beispiel 5e beschriebenen Vorschrift durch Umsetzung von 0,84g 3,4-Diaminothiophen Dihydrobromid mit 0,8 g 2,4-Dichlor-3-thienyl-isothiocyanat und 0,49g N-Ethyl-N,N-diisopropylamin in THF. Kristallines Produkt. Schmp. 110-115°C.

### k) 2-(2,4-Dichlor-3-thienylamino)-1 H-thieno[3,4-d]imidazol Hydrochlorid

Zu einer Mischung, dargestellt aus einer Lösung von 157 mg N-(4-Amino-3-thienyl)-N`-(2,4-dichlorthienyl)thioharnstoff in 15 ml THF und einer Lösung aus 51,08 mg NaOH in 2ml Wasser, tropfte man eine Lösung aus 108,4 mg p-Toluolsulfonsäurechlorid in 5 ml wasserfreiem THF. Die erhaltene dunkle Lösung wurde 2 Stunden bei Raumtempeatur gerührt, das Lösungsmittel abdestilliert und der Rückstand mit Wasser versetzt. Nach Extraktion mit Ethylacetat wusch man die organische Phase mit Wasser und trocknete sie über Natriumsulfat. Nach dem Abdestillieren des Lösungsmittels reinigte man durch Säulenchromatografie an Kieselgel mit einem Gemisch von 20 Volumenteilen Ethylacetat, 10 Volumenteilen n-Heptan, 3 Volumenteilen Eisessig als Elutionsmittel, das sodann unter vermindertem Druck abdestilliert wurde. Man erhielt ein viskoses amorphes Produkt, das in Ethylacetat gelöst und mit Chlorwasserstoffgas-gesättigtem Diethylether stark sauer gestellt wurde. Das sich abscheidende amorphe salzsaure Salz wurde durch Anreiben zur Kristallisation gebracht. Schmp.: > 300°C.

### Beispiel 11: 2-(2-Chlor-4-methyl-3-thienylamino)-1 H-thieno[3,4-d]imidazol

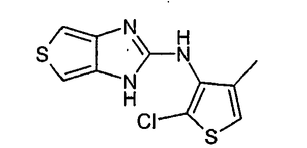

erhält man durch Zugabe von gesättigter Natriumhydrogencarbonat-Lösung zu einer Suspension von 306 mg 2-(2-Chlor-4-methyl-3-thienylamino)-1 H-th ieno[3,4-d]imidazol Hydrochlorid (Beispiel 5) in 40 ml Wasser, wobei ein pH von etwa 1 0 eingestellt wird. Nach dem Rühren der Suspension über 1 Stunde bei Raumtemperatur filtriert man den Feststoff ab, wäscht ihn mehrfach mit Wasser und trocknet das Produkt im Luftstrom. Farbloses kristallines Produkt, Zersetzungspunkt ab 180°C.

### Pharmakologische Daten:

### Bestimmung der NHE-Hemmung

### Testbeschreibung:

In diesem Test wurde die Erholung des intrazellulären pHs (pHᵢ) nach einer Ansäuerung ermittelt, die bei funktionsfähigem NHE auch unter bicarbonatfreien Bedingungen einsetzt. Dazu wurde der pHᵢ mit dem pH-sensitiven Fluoreszenzfarbstoff BCECF (Calbiochem, eingesetzt wird die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Die Zellen wurden bereits bei der BCECF-Beladung in NH₄Cl-Puffer (pH 7,4) inkubiert (NH₄Cl-Puffer: 115 mM NaCl, 20 mM NH₄Cl, 5 mM KCl, 1 mM CaCl₂, 1 mM MgSO₄, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines NH₄Cl -freien Puffers (s. u.) zu 25 µl Aliquots der in NH₄Cl-Puffer inkubierten Zellen induziert. Die nachfolgende Geschwindigkeit der pH-Erholung wurde bei NHE1 zwei Minuten, bei NHE2 fünf Minuten und bei NHE3 drei Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCI, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wird ein pH von 7,0 eingestellt). Für die Bestimmung des 0%-Wertes wurden die Zellen in einem Na⁺-freien Puffer inkubiert (133,8 mM Cholinchlorid, 4,7 mM KCI, 1,25 mM CaCl₂, 1,25 mM MgCl₂, 0,97 mM K₂HPO₄, 0,23 mM KH₂PO₄, 5 mM Hepes, 5 mM Glucose, mit 1 M KO H wird ein pH von 7,0 eingestellt). Die zu testenden Substanzen wurden in dem Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms Sigma-Plot der IC₅₀-Wert der jeweiligen Substanz für die einzelnen NHE-Subtypen berechnet.

### Ergebnisse:

| Beispiel | IC₅₀ (NHE3) |
|---|---|
| Beispiel 2 | 0,22 µM |
| Beispiel 5 | 0;15 µM |
| Beispiel 6 | 6,51 µM |
| Beispiel 9 | 1,43 µM |
| Beispiel 10 | 0,32 µM |

## Patentansprüche

1. Verbindungen der Formeln oder II, 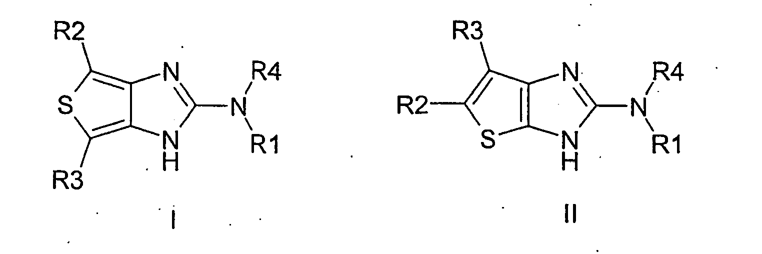 worin bedeuten
R1 Phenyl, das in 2- und 6-Stellung mit den Resten R5 und R6 substituiert ist;
R5 und R6 unabhängig voneinander Wasserstoff, Methyl, Ethyl, Cycloalkyl mit 3, 4 oder 5 C-Atomen, Vinyl, Ethinyl, Fluor, Chlor, Brom, lod, Cyano, Trifluormethyl, SF₅, Methoxy, Nitro oder -X-R7;
R7 Alkyl mit 1, 2 oder 3 C-Atomen, Trifluormethyl oder CH₂-CF₃;
X CH₂, O, NH oder S(O)ₙ;
n Null, eins oder zwei;
wobei R5 und R6 nicht beide gleichzeitig Wasserstoff entsprechen; oder
R1 3-Thienyl das in 2- und 4-Stellung mit den Resten R5 und R6 substituiert ist;
R5 und R6 unabhängig voneinander Wasserstoff, Methyl, Ethyl, Cycloalkyl mit 3, 4 oder 5 C-Atomen, Vinyl, Ethinyl, Fluor, Chlor, Brom, lod, CN, NO₂, Trifluormethyl, SF₅, Methoxy oder -X-R7;
R7 Alkyl mit 1, 2 oder 3 C-Atomen, Trifluormethyl oder CH₂-CF₃;
X CH₂, O, NH, S(O)ₙ;
n Null, eins oderzwei;
wobei R5 und R6 nicht beide gleichzeitig Wasserstoff entsprechen;
R2 und R3 unabhängig voneinander Wasserstoff, Fluor, Chlor, Brom, Methyl, CN, OH, -O- CH₃, NO₂, NH₂, -CF₃, oder -Y-R8;
R8 Methyl, Trifluormethyl oder CH₂-CF₃;
Y CH₂, O, NH oder S(O)ₘ;
m Null, eins oder zwei,
R4 Wasserstoff, M ethyl, Ethyl, Cyclopropyl, Cyclopropylmethyl oder CH₂-CF₃;
sowie deren pharmazeutisch verträgliche Salze und deren Trifluoressigsäuresalze.

2. Verbindungen der Formel I nach Anspruch 1,
worin bedeuten
R1 Phenyl, das in 2- und 6-Stellung mit den Resten R5 und R6 substituiert ist;
R5 und R6 unabhängig voneinander Wasserstoff, Methyl, Ethyl, Cycloalkyl mit 3, 4 oder 5 C-Atomen, Vinyl, Ethinyl, Fluor, Chlor, Brom, lod, Cyano, Trifluormethyl, SF₅, Methoxy, Nitro oder -X-R7;
R7 Alkyl mit 1, 2 oder 3 C-Atomen, Trifluormethyl oder CH₂-CF₃;
X CH₂, O, NH oder S(O)ₙ;
n Null, eins oder zwei;
wobei R5 und R6 nicht beide gleichzeitig Wasserstoff entsprechen; oder
R1 3-Thienyl das in 2- und 4-Stellung mit den Resten R5 und R6 substituiert ist;
R5 und R6 unabhängig voneinander Wasserstoff, Methyl, Ethyl, Cycloalkyl mit 3, 4 oder 5 C-Atomen, Vinyl, Ethinyl, Fluor, Chlor, Brom, lod, CN, NO₂, Trifluormethyl, SF₅, Methoxy oder -X-R7;
R7 Alkyl mit 1, 2 oder 3 C-Atomen, Trifluormethyl oder CH₂-CF₃;
X CH₂, O, NH, S(O)ₙ;
n Null, eins oder zwei;
wobei R5 und R6 nicht beide gleichzeitig Wasserstoff entsprechen;
R2 und R3 unabhängig voneinander Wasserstoff, Fluor, Chlor, Brom, Methyl, CN, OH, -O-CH₃, NO₂, NH₂, -CF₃, oder-Y-R8;
R8 Methyl, Trifluormethyl oder CH₂-CF₃;
Y CH₂, O, NH oder S(O)ₘ;
m Null, eins oder zwei,
R4 Wasserstoff, Methyl, Ethyl, Cyclopropyl, Cyclopropylmethyl oder CH₂-CF₃;
sowie deren pharmazeutisch verträgliche Salze und deren Trifluoressigsäuresalze.

3. Verbindungen der Formel I nach Anspruch 1 oder 2,
worin bedeuten
R1 Phenyl, das in 2- und 6-Stellung mit den Resten R5 und R6 substituiert ist;
R5 und R6 unabhängig voneinander Wasserstoff, Methyl, Ethyl, Fluor, Chlor, Brom oder Trifluormethyl;
wobei R5 und R6 nicht beide gleichzeitig Wasserstoff entsprechen; oder
R1 3-Thienyl das in 2- und 4-Stellung mit den Resten R5 und R6 substituiert ist;
R5 und R6 unabhängig voneinander Wasserstoff, Methyl, Ethyl, Fluor, Chlor, Brom oder Trifluormethyl;
wobei R5 und R6 nicht beide gleichzeitig Wasserstoff entsprechen;
R2, R3 und R4 Wasserstoff
sowie deren pharmazeutisch verträgliche Salze und deren Trifluoressigsäuresalze.

4. Verbindungen der Formel I nach einem oder mehreren Ansprüchen 1, 2 und 3, ausgewählt aus der Gruppe:
2-(4-Methyl-3-thienylamino)-1H-thieno[3,4-d]imidazol,
2-(2,6-Dichlorphenylamino)-1H-thieno[3,4-d]imidazol,
2-(2-Chlor-4-methyl-3-thienylamino)-1H-thieno[3,4-d]imidazol,
2-(2 -Trifluormethylphenylamino)-1H-thieno[3,4-d]imidazol,
2-(2,6 -Dimethylphenylamino)-1H-thieno[3,4-d]imidazol,
2-(2,6 -Difluorphenylamino)-1H-thieno[3,4-d]imidazol,
2-(2-Chlor-6-methylphenylamino)-1H-thieno[3,4-d]imidazol,
2-(2,4-Dichlor-3-thienylamino)-1H-thieno[3,4-d]imidazol
und
2-(2-Chlor-4-methyl-3-thienylamino)-1H-thieno[3,4-d]imidazol,
sowie deren pharmazeutisch verträgliche Salze und deren Trifluoressigsäuresalze.

5. Verbindungen der Formel I nach einem oder mehreren Ansprüchen 1, 2, 3 und 4, ausgewählt aus der Gruppe:
2-(2,6-Dichlorphenylamino)-1 H-thieno[3,4-d]imidazol,
2-(2-Chlor-4-methyl-3-thienylamino)-1 H-thieno[3,4-d]imidazol
und
2-(2,4-Dichlor-3-thienylamino)-1 H-thieno[3,4-d]imidazol,
sowie deren pharmazeutisch verträgliche Salze und deren Trifluoressigsäuresalze.

6. Verbindungen der Formel I oder II oder deren pharmazeutisch verträgliche Salze nach einem oder mehreren der Ansprüche 1 bis 5 zur Verwendung als Medikament.

7. Verwendung einer Verbindung der Formel 1 oder 11 oder deren pharmazeutisch verträglicher Salze nach einem oder mehreren der Ansprüche 1 bis 5 zur Herstellung eines Medikaments zur Behandlung oder Prophylaxe von Störungen des Atemantriebs, von schlafbedingten Atemstörungen, Schlafapnoen, oder des Schnarchens, zur Behandlung oder zur Prophylaxe von akuten und chronischen Nierenerkrankungen, des akuten Nierenversagens oder des chronischen Nierenversagens, oder von Störungen der Darmfunktion, zur Behandlung oder Prophylaxe des Bluthochdrucks, der essentiellen Hypertonie, von Erkrankungen des Zentralnervensystems, von Erkrankungen, die durch ZNS-Übererregbarkeit resultieren, Epilepsie oder zentral ausgelöste Krämpfe, und von Erkrankungen des Zentralnervensystems, von Angstzuständen, Depressionen und Psychosen, zur Behandlung oder Prophylaxe von akuten und chronischen Schäden, Erkrankungen und indirekte Folgeerkrankungen von Organen oder Geweben, die durch Ischämie- oder durch Reperfusionsereignisse verursacht werden, von Arrhythmien, von Atherosklerose, von Hypercholesterinämie, von Krankheiten; bei denen die Zellproliferation eine primäre oder sekundäre Ursache darstellt, von Krebs, von Prostata-Hypertrophie bzw. Prostata-Hyperplasie, von fibrotischen Erkrankungen innerer Organe, von Herzinsuffizienz oder von Congestive Heart Failure, von Thrombosen, von Störungen der Gallenfunktion, des Befalls durch Ektoparasiten, von Erkrankungen infolge endothelialer Dysfunktion, von claudicatio intermittens, von Protozoen-Erkrankungen, der Malaria, zur Behandlung von Schockzuständen, der Zuckerkrankheit und diabetischer Spätschäden, akuter oder chronischer inflammatorischer Erkrankungen oder zur Herstellung eines Medikaments zur Konservierung und Lagerung von Transplantaten für chirurgische Maßnahmen, zur Herstellung eines Medikaments für den Einsatz bei chirurgischen Operationen und Organtransplantationen, zur Herstellung eines Medikaments zur Verhinderung altersbedingter Gewebsveränderung, zur Gesunderhaltung und Lebensverlängerung, oder zur Herstellung eines Medikaments zur Verminderung cytotoxischer Effekte oder zur Verwendung als Diagnostika.

8. Verwendung einer Verbindung der Formel I oder II oder deren pharmazeutisch verträgliche Salze nach einem oder mehreren der Ansprüche 1 bis 5 in Kombination mit einem oder mehreren anderen Arzneimitteln oder Wirkstoffen zur Herstellung eines Medikaments zur Behandlung oder Prophylaxe von Störungen des Atemantriebs, von schlafbedingten Atemstörungen, Schlafapnoen, oder des Schnarchens, zur Behandlung oder zur Prophylaxe von akuten und chronischen Nierenerkrankungen, des akuten Nierenversagens oder des chronischen Nierenversagens, oder von Störungen der Darmfunktion, zur Behandlung oder Prophylaxe des Bluthochdrucks, der essentiellen Hypertonie, von Erkrankungen des Zentralnervensystems, von Erkrankungen, die durch ZNS-Übererregbarkeit resultieren, Epilepsie oder zentral ausgelöste Krämpfe, und von Erkrankungen des Zentralnervensystems, von Angstzuständen, Depressionen und Psychosen, zur Behandlung oder Prophylaxe von akuten und chronischen Schäden, Erkrankungen und indirekte Folgeerkrankungen von Organen oder Geweben, die durch Ischämie- oder durch Reperfusionsereignisse verursacht werden, von Arrhythmien, von Atherosklerose, von Hypercholesterinämie, von Krankheiten, bei denen die Zellproliferation eine primäre oder sekundäre Ursache darstellt, von Krebs, von Prostata-Hypertrophie bzw: Prostata-Hyperplasie, von fibrotischen Erkrankungen innerer Organe, von Herzinsuffizienz oder von Congestive Heart Failure, von Thrombosen, von Störungen der Gallenfunktion, des Befalls durch Ektoparasiten, von Erkrankungen infolge endothelialer Dysfunktion, von claudicatio intermittens, von Protozoen-Erkrankungen, der Malaria, zur Behandlung von Schockzuständen, der Zuckerkrankheit und diabetischer Spätschäden, akuter oder chronischer inflammatorischer Erkrankungen oder zur Herstellung eines Medikaments zur Konservierung und Lagerung von Transplantaten für chirurgische Maßnahmen, zur Herstellung eines Medikaments für den Einsatz bei chirurgischen Operationen und Organtransplantationen, zur Herstellung eines Medikaments zur Verhinderung altersbedingter Gewebsveränderung, zur Gesunderhaltung und Lebensverlängerung oder zur Herstellung eines Medikaments zur Verminderung cytotoxischer Effekte.

9. Verwendung einer Verbindung der Formel I oder II oder deren pharmazeutisch verträgliche Salze nach einem oder mehreren der Ansprüche 1 bis 5 allein oder in Kombination mit anderen Arzneimitteln oder Wirkstoffen zur Herstellung eines Medikaments zur Behandlung oder Prophylaxe von Störungen des Atemantriebs, von Schlaf-bedingten Atemstörungen oder Schlafapnoen.

10. Verwendung einer Verbindung der Formel I oder II oder deren pharmazeutisch verträgliche Salze nach einem oder mehreren der Ansprüche 1 bis 5 allein oder in Kombination mit anderen Arzneimitteln oder Wirkstoffen zur Herstellung eines Medikaments zur Behandlung oder Prophylaxe des Schnarchens.

11. Verwendung einer Verbindung der Formel I oder II oder deren pharmazeutisch verträgliche Salze nach einem oder mehreren der Ansprüche 1 bis 5 allein oder in Kombination mit anderen Arzneimitteln oder Wirkstoffen zur Herstellung eines Medikaments zur Behandlung oder zur Prophylaxe von akuten und chronischen Nierenerkrankungen, des akuten Nierenversagens oder des chronischen Nierenversagens.

12. Verwendung einer Verbindung der Formel I oder II oder deren pharmazeutisch verträgliche Salze nach einem oder mehreren der Ansprüche 1 bis 5 allein oder in Kombination mit anderen Arzneimitteln oder Wirkstoffen zur Herstellung eines Medikaments zur Behandlung oder Prophylaxe von Störungen der Darmfunktion.

13. Heilmittel für die humane, veterinäre oder phytoprotektive Anwendung, enthaltend eine wirksame Menge mindestens einer Verbindung der Formel I oder II und/oder deren pharmazeutisch verträglicher Salze nach einem oder mehreren der Ansprüche 1 bis 5, zusammen mit pharmazeutisch annehmbaren Träger- und Zusatzstoffen.

14. Heilmittel für die humane, veterinäre oder phytoprotektive Anwendung, enthaltend eine wirksame Menge mindestens einer Verbindung der Formel I oder II und/oder deren pharmazeutisch verträglicher Salze nach einem oder mehreren der Ansprüche 1 bis 5, zusammen mit pharmazeutisch annehmbaren Träger-und Zusatzstoffen, in Kombination mit anderen pharmakologischen Wirkstoffen oder Arzneimitteln.

15. Verfahren zur Herstellung einer Verbindung der Formeln I oder II und/oder deren pharmazeutisch verträglicher Salze, **dadurch gekennzeichnet, dass** man
a) ein Diamin der Formeln III oder IV mit einem Cyanat der Formel V zu einem Harnstoff-Derivat der Formel VI oder VII umsetzt
b) das Harnstoff-Derivat der Formel VI oder VII zu einer Verbindung der Formeln la oder IIa cyclisiert
und
c) zur Herstellung einer Verbindung der Formeln I oder II, in der R4 verschieden von Wasserstoff ist, eine Verbindung der Formeln la oder lla zu einer Verbindung der Formeln I oder II derivatisiert, 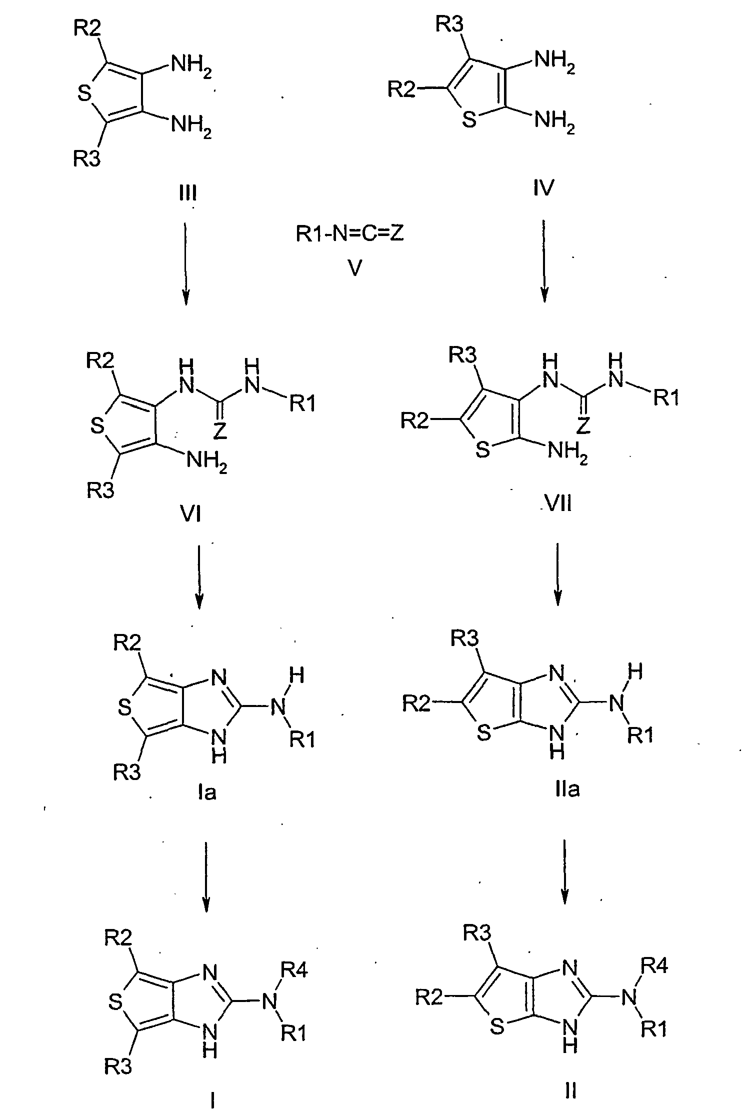
worin R1, R2, R3 und R4 die in den Ansprüchen 1 bis 5 angegebene Bedeutung besitzen und wobei Z Sauerstoff oder bevorzugt Schwefel ist.

## Claims

1. A compound of the formula I or II 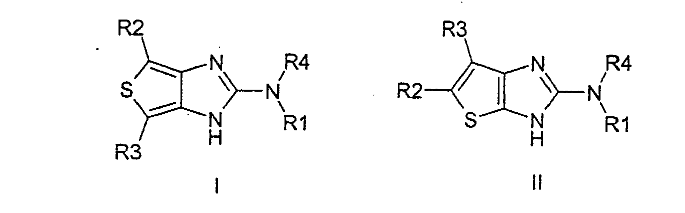 where
R1 is phenyl which is substituted by the radicals R5 and R6 in the 2-and 6-position;
R5 and R6 are each independently hydrogen, methyl, ethyl, cycloalkyl having 3, 4 or 5 carbon atoms, vinyl, ethynyl, fluorine, chlorine, bromine, iodine, cyano, trifluoromethyl, SF₅, methoxy, nitro or -X-R7;
R7 is alkyl having 1, 2 or 3 carbon atoms, trifluoromethyl or CH₂-CF₃;
X is CH₂, O, NH or S(O)ₙ;
n is zero, one or two;
where R5 and R6 are not both at the same time hydrogen;
or
R1 is 3-thienyl which is substituted by the radicals R5 and R6 in the 2-and 4-position;
R5 and R6 are each independently hydrogen, methyl, ethyl, cycloalkyl having 3, 4 or 5 carbon atoms, vinyl, ethynyl, fluorine, chlorine, bromine, iodine, CN, NO₂, trifluoromethyl, SF₅, methoxy or -X-R7;
R7 is alkyl having 1, 2 or 3 carbon atoms, trifluoromethyl or CH₂-CF₃;
X is CH₂, O, NH or S(O)ₙ;
n is zero, one or two;
where R5 and R6 are not both at the same time hydrogen;
R2 and R3 are each independently hydrogen, fluorine, chlorine, bromine, methyl, CN, OH, -O-CH₃, NO₂, NH₂, -CF₃ or-Y-R8;
R8 is methyl, trifluoromethyl or CH₂-CF₃;
Y is CH₂, 0, NH or S(O)ₘ;
m is zero, one or two,
R4 is hydrogen, methyl, ethyl, cyclopropyl, cyclopropylmethyl or CH₂-CF₃;
and a pharmaceutically acceptable salt and a trifluoroacetic acid salt thereof.

2. A compound of the formula I as claimed in claim 1,
where
R1 is phenyl which is substituted by the radicals R5 and R6 in the 2-and 6-position;
R5 and R6 are each independently hydrogen, methyl, ethyl, cycloalkyl having 3, 4 or 5 carbon atoms, vinyl, ethynyl, fluorine, chlorine, bromine, iodine, cyano, trifluoromethyl, SF₅, methoxy, nitro or -X-R7;
R7 is alkyl having 1, 2 or 3 carbon atoms, trifluoromethyl or CH₂-CF₃;
X is CH₂, O, NH or S(O)ₙ;
n is zero, one or two;
where R5 and R6 are not both at the same time hydrogen;
or
R1 is 3-thienyl which is substituted by the radicals R5 and R6 in the 2-and 4-position;
R5 and R6 are each independently hydrogen, methyl, ethyl, cycloalkyl having 3, 4 or 5 carbon atoms, vinyl, ethynyl, fluorine, chlorine, bromine, iodine, CN, NO₂, trifluoromethyl, SF₅, methoxy or -X-R7;
R7 is alkyl having 1, 2 or 3 carbon atoms, trifluoromethyl or CH₂-CF₃;
X is CH₂, O, NH or S(O)ₙ;
n is zero, one or two;
where R5 and R6 are not both at the same time hydrogen;
R2 and R3 are each independently hydrogen, fluorine, chlorine, bromine, methyl, CN, OH, -O-CH₃, NO₂, NH₂, -CF₃ or -Y-R8;
R8 is methyl, trifluoromethyl or CH₂-CF₃;
Y is CH₂, O, NH or S(O)ₘ;
m is zero, one or two,
R4 is hydrogen, methyl, ethyl, cyclopropyl, cyclopropylmethyl or CH₂-CF₃; and a pharmaceutically acceptable salt and a trifluoroacetic acid salts thereof.

3. A compound of the formula I as claimed in claim 1 or 2
where
R1 is phenyl which is substituted by the radicals R5 and R6 in the 2-and 6-position;
R5 and R6 are each independently hydrogen, methyl, ethyl, fluorine, chlorine, bromine or trifluoromethyl;
where R5 and R6 are not both at the same time hydrogen;
or
R1 is 3-thienyl which is substituted by the radicals R5 and R6 in the 2-and 4-position;
R5 and R6 are each independently hydrogen, methyl, ethyl, fluorine, chlorine, bromine or trifluoromethyl;
where R5 and R6 are not both at the same time hydrogen;
R2, R3 and R4 are each hydrogen
and a pharmaceutically acceptable salt and a trifluoroacetic acid salt thereof.

4. A compound of the formula I as claimed in one or more of claims 1, 2 and 3, selected from the group of:
2-(4-methyl-3-thienylamino)-1 H-thieno[3,4-d]imidazole,
2-(2,6-dichlorophenylamino)-1 H-thieno[3,4-d]imidazole,
2-(2-chloro-4-methyl-3-thienylamino)-1 H-thieno[3,4-d]imidazole,
2-(2-trifluoromethylphenylamino)-1 H-thieno[3,4-d]imidazole,
2-(2,6-dimethylphenylamino)-1 H-thieno[3,4-d]imidazole,
2-(2,6-difluorophenylamino)-1 H-thieno[3,4-d]imidazole,
2-(2-chloro-6-methylphenylamino)-1 H-thieno[3,4-d]imidazole,
2-(2,4-dichloro-3-thienylamino)-1 H-thieno[3,4-d]imidazole and
2-(2-chloro-4-methyl-3-thienylamino)-1 H-thieno[3,4-d]imidazole,
and a pharmaceutically acceptable salt and a trifluoroacetic acid salts thereof.

5. A compound of the formula I as claimed in one or more of claims 1, 2, 3 and 4, selected from the group of:
2-(2,6-dichlorophenylamino)-1H-thieno[3,4-d]imidazole,
2-(2-chloro-4-methyl-3-thienylamino)-1 H-thieno[3,4-d]imidazole and
2-(2,4-dichloro-3-thienylamino)-1 H-thieno[3,4-d]imidazole,
and a pharmaceutically acceptable salt and a trifluoroacetic acid salt thereof.

6. A compound of the formula I or II or a pharmaceutically acceptable salt thereof as claimed in one or more of claims 1 to 5 for use as a medicament.

7. The use of a compound of the formula I or 11 or of its pharmaceutically acceptable salts as claimed in one or more of claims 1 to 5 for preparing a medicament for the treatment or prophylaxis of disorders of respiratory drive, sleep-related respiratory disorders, sleep apneas, or of snoring, for the treatment or for the prophylaxis of acute and chronic renal disorders, of acute renal failure or of chronic renal failure, or of intestinal dysfunction, or for the treatment or prophylaxis of high blood pressure, of essential hypertension, of disorders of the central nervous system, of disorders which result from CNS hyperexcitability, epilepsy or centrally induced convulsions, and of disorders of the central nervous system, of anxiety states, depressions and psychoses, for the treatment or prophylaxis of acute and chronic damage to, disorders of and indirect sequelae of organs or tissues which are caused by ischemic or by reperfusion events, of arrhythmias, of atherosclerosis, of hypercholesterolemia, of diseases for which cell proliferation constitutes a primary or secondary cause, of cancer, of prostate hypertrophy and prostate hyperplasia, of fibrotic disorders of internal organs, of heart failure or of congestive heart failure, of thromboses, of disorders of biliary function, of infestation by ectoparasites, of disorders resulting from endothelial dysfunction, of intermittent claudication, of protozoal disorders, of malaria, for the treatment of shock, of diabetes and late damage from diabetes, acute or chronic inflammatory disorders or for the preparation of a medicament for preserving and storing transplants for surgical measures, for the preparation of a medicament for use in surgical operations and organ transplants, for the preparation of a medicament for preventing age-related tissue change, for preserving health and prolonging life, or for the preparation of a medicament for reducing cytotoxic effects or for use as a diagnostic aid.

8. The use of a compound of the formula I or II or of its pharmaceutically acceptable salts as claimed in one or more of claims 1 to 5 in combination with one or more other medicaments or active ingredients for preparing a medicament for the treatment or prophylaxis of disorders of respiratory drive, sleep-related respiratory disorders, sleep apneas, or of snoring, for the treatment or for the prophylaxis of acute and chronic renal disorders, of acute renal failure or of chronic renal failure, or of intestinal dysfunction, or for the treatment or prophylaxis of high blood pressure, of essential hypertension, of disorders of the central nervous system, of disorders which result from CNS hyperexcitability, epilepsy or centrally induced convulsions, and of disorders of the central nervous system, of anxiety states, depressions and psychoses, for the treatment or prophylaxis of acute and chronic damage to, disorders of and indirect sequelae of organs or tissues which are caused by ischemic or by reperfusion events, of arrhythmias, of atherosclerosis, of hypercholesterolemia, of diseases for which cell proliferation constitutes a primary or secondary cause, of cancer, of prostate hypertrophy and prostate hyperplasia, of fibrotic disorders of internal organs, of heart failure or of congestive heart failure, of thromboses, of biliary dysfunction, of infestation by ectoparasites, of disorders resulting from endothelial dysfunction, of intermittent claudication, of protozoal disorders, of malaria, for the treatment of shock, of diabetes and late damage from diabetes, acute or chronic inflammatory disorders or for the preparation of a medicament for preserving and storing transplants for surgical measures, for the preparation of a medicament for use in surgical operations and organ transplants, for the preparation of a medicament for preventing age-related tissue change, for preserving health and prolonging life, or for the preparation of a medicament for reducing cytotoxic effects.

9. The use of a compound of the formula I or II or of a pharmaceutically acceptable salt thereof as claimed in one or more of claims 1 to 5, alone or in combination with other medicaments or active ingredients, for preparing a medicament for the treatment or prophylaxis of impairment of respiratory drive, of sleep-related respiratory disturbances or sleep apneas.

10. The use of a compound of the formula I or II or of a pharmaceutically acceptable salt thereof as claimed in one or more of claims 1 to 5, alone or in combination with other medicaments or active ingredients, for preparing a medicament for the treatment or prophylaxis of snoring.

11. The use of a compound of the formula I or II or of a pharmaceutically acceptable salt thereof as claimed in one or more of claims 1 to 5, alone or in combination with other medicaments or active ingredients, for preparing a medicament for the treatment or for the prophylaxis of acute and chronic renal disorders, of acute renal failure or of chronic renal failure.

12. The use of a compound of the formula I or II or of a pharmaceutically acceptable salt thereof as claimed in one or more of claims 1 to 5, alone or in combination with other medicaments or active ingredients, for preparing a medicament for the treatment or prophylaxis of intestinal dysfunction.

13. A curative composition for human, veterinary or phytoprotective use, comprising an effective amount of at least one compound of the formula I or II and/or a pharmaceutically acceptable salt thereof as claimed in one or more of claims 1 to 5, together with pharmaceutically acceptable carriers and additives.

14. A curative composition for human, veterinary or phytoprotective use, comprising an effective amount of at least one compound of the formula I or II and/or a pharmaceutically acceptable salt thereof as claimed in one or more of claims 1 to 5, together with pharmaceutically acceptable carriers and additives, in combination with other pharmacological active ingredients or medicaments.

15. A process for preparing a compound of the formulae I or II and/or a pharmaceutically acceptable salt thereof, which comprises
a) reacting a diamine of the formulae III or IV with a cyanate of the formula V to give a urea derivative of the formula VI or VII,
b) cyclizing the urea derivative of the formula VI or VII to give a compound of the formulae Ia or IIa,
and
c) to prepare a compound of the formulae I or II in which R4 is other than hydrogen, derivatizing a compound of the formulae la or IIa to give a compound of the formulae I or II 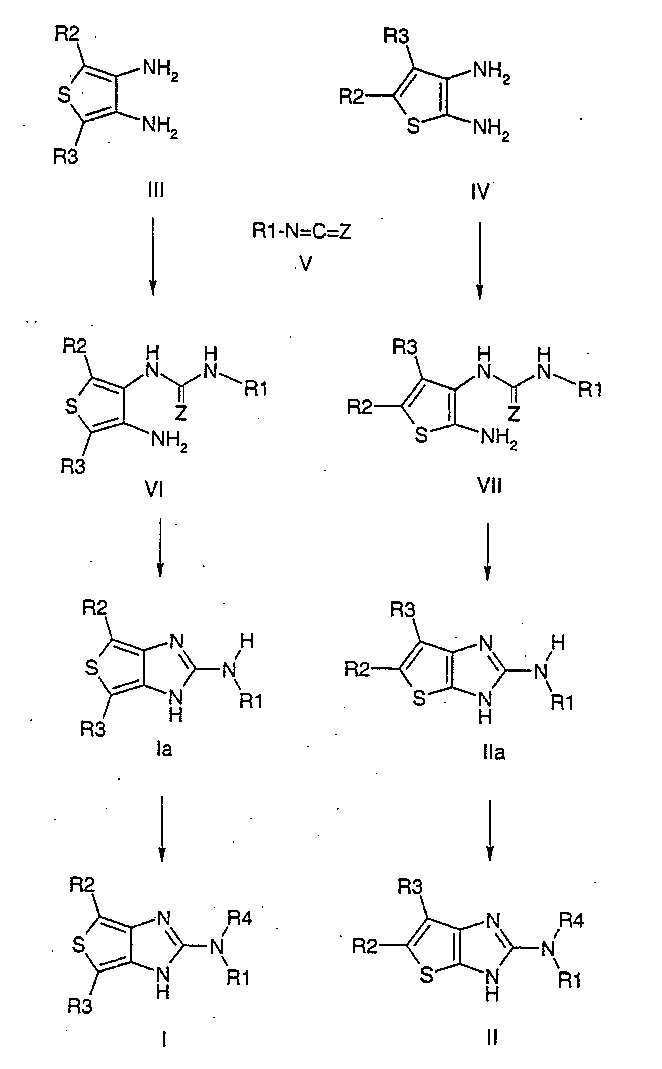
where R1, R2, R3 and R4 are each as defined in claims 1 to 5, and where Z is oxygen or preferably sulfur.

## Revendications

1. Composés de formule I ou II 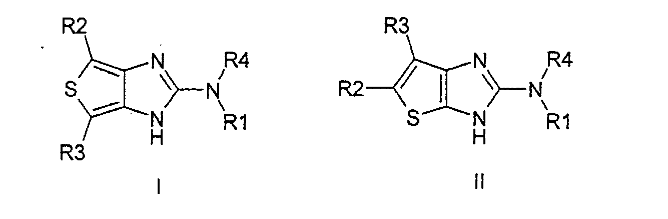 dans laquelle
R1 est phényle qui est substitué par les radicaux R5 et R6 en position 2 et 6;
R5 et R6 sont chacun indépendamment hydrogène, méthyle, éthyle, cycloalkyle ayant 3, 4 ou 5 atomes de carbone, vinyle, éthynyle, fluor, chlore, brome, iode, cyano, trifluorométhyle, SF₅, méthoxy, nitro ou -X-R7 ;
R7 est alkyle ayant 1, 2 ou 3 atomes de carbone, trifluorométhyle ou CH₂-CF₃ ;
X est CH₂, O, NH ou S(O)ₙ ;
n vaut zéro, un ou deux ;
où R5 et R6 ne sont pas tous les deux en même temps hydrogène ;
ou
R1 est 3-thiényle qui est substitué par les radicaux R5 et R6 en position 2et4;
R5 et R6 sont chacun indépendamment hydrogène, méthyle, éthyle, cycloalkyle ayant 3, 4 ou 5 atomes de carbone, vinyle, éthynyle, fluor, chlore, brome, iode, CN, NO₂, trifluorométhyle, SF₅, méthoxy ou -X-R7;
R7 est alkyle ayant 1, 2 ou 3 atomes de carbone, trifluorométhyle ou CH₂-CF₃ ;
X est CH₂, O, NH ou S(O)ₙ ;
n vaut zéro, un ou deux ;
où R5 et R6 ne sont pas tous les deux en même temps hydrogène ;
R2 et R3 sont chacun indépendamment hydrogène, fluor, chlore, brome, méthyle, CN, OH, -O-CH₃, NO₂, NH₂, -CF₃ ou -Y-R8 ;
R8 est méthyle, trifluorométhyle ou CH₂-CF₃ ;
Y est CH₂, O, NH ou S(O)ₘ;
m vaut zéro, un ou deux,
R4 est hydrogène, méthyle, éthyle, cyclopropyle, cyclopropylméthyle ou CH₂-CF₃ ;
et sels pharmaceutiquement acceptables et sels d'acide trifluoracétique de ceux-ci.

2. Composés de formule I selon la revendication 1,
dans laquelle
R1 est phényle qui est substitué par les radicaux R5 et R6 en position 2 et 6 ;
R5 et R6 sont chacun indépendamment hydrogène, méthyle, éthyle, cycloalkyle ayant 3, 4 ou 5 atomes de carbone, vinyle, éthynyle, fluor, chlore, brome, iode, cyano, trifluorométhyle, SF₅, méthoxy, nitro ou -X-R7 ;
R7 est alkyle ayant 1, 2 ou 3 atomes de carbone,trifluorométhyle ou CH₂-CF₃ ;
X est CH₂, O, NH ou S(O)ₙ ;
n vaut zéro, un ou deux ;
où R5 et R6 ne sont pas tous les deux en même temps hydrogène ;
ou
R1 est 3-thiényle qui est substitué par les radicaux R5 et R6 en position 2 et 4;
R5 et R6 sont chacun indépendamment hydrogène, méthyle, éthyle, cycloalkyle ayant 3, 4 ou 5 atomes de carbone, vinyle, éthynyle, fluor, chlore, brome, iode, CN, NO₂, trifluorométhyle, SF₅, méthoxy ou -X-R7;
R7 est alkyle ayant 1, 2 ou 3 atomes de carbone, trifluorométhyle ou CH₂-CF₃;
X X est CH₂, O, NH ou S(O)ₙ ;
n vaut zéro, un ou deux ;
où R5 et R6 ne sont pas tous les deux en même temps hydrogène ;
R2 et R3 sont chacun indépendamment hydrogène, fluor, chlore, brome, méthyle, CN, OH, -O-CH₃, NO₂, NH₂, -CF₃ ou -Y-R8 ;
R8 est méthyle, trifluorométhyle ou CH₂-CF₃ ;
Y est CH₂, O, NH ou S(O)ₘ;
m vaut zéro, un ou deux,
R4 est hydrogène, méthyle, éthyle, cyclopropyle, cyclopropylméthyle ou CH₂-CF₃ ;
et sels pharmaceutiquement acceptables et sels d'acide trifluoracétique de ceux-ci.

3. Composés de formule 1 selon la revendication 1 ou 2,
dans laquelle
R1 est phényle qui est substitué par les radicaux R5 et R6 en position 2 et 6 ;
R5 et R6 sont chacun indépendamment hydrogène, méthyle, éthyle, fluor, chlore, brome ou trifluorométhyle ;
où R5 et R6 ne sont pas tous les deux en même temps hydrogène ;
ou
R1 est 3-thiényle qui est substitué par les radicaux R5 et R6 en position 2et4;
R5 et R6 sont chacun indépendamment hydrogène, méthyle, éthyle, fluor, chlore, brome ou trifluorométhyle ;
où R5 et R6 ne sont pas tous les deux en même temps hydrogène ;
R2, R3 et R4 sont chacun hydrogène
et sels pharmaceutiquement acceptables et sels d'acide trifluoracétique de ceux-ci.

4. Composés de formule I selon une ou plusieurs parmi les revendications 1, 2 et 3, choisi parmi le groupe constitué par :
le 2-(4-méthyl-3-thiénylamino)-1H-thiéno[3,4-d]imidazole,
le 2-(2,6-dichlorophénylamino)-1H-thiéno[3,4-d]imidazole,
le 2-(2-chloro-4-méthyl-3-thiénylamino)-1 H-thiéno[3,4-d]imidazole,
le 2-(2-trifluorométhylphénylamino)-1H-thiéno[3,4-d]imidazole,
le 2-(2,6-diméthylphénylamino)-1H-thiéno[3,4-d]imidazole,
le 2-(2,6-difluorophénylamino)-1H-thiéno[3,4-d]imidazole,
le 2-(2-chloro-6-méthylphénylamino)-1H-thiéno[3,4-d]imidazole,
le 2-(2,4-dichloro-3-thiénylamino)-1H-thiéno[3,4-d]imidazole
et
le 2-(2-chloro-4-méthyl-3-thiénylamino)-1 H-thiéno[3,4-d]imidazole,
et sels pharmaceutiquement acceptables et sels d'acide trifluoracétique de ceux-ci.

5. Composés de formule I selon une ou plusieurs parmi les revendications 1, 2, 3 et 4, choisi parmi le groupe constitué par :
le 2-(2,6-dichlorophénylamino)-1H-thiéno[3,4-d]imidazole,
le 2-(2-chloro-4-méthyl-3-thiénylamino)-1 H-thiéno[3,4-d]imidazole
et
le 2-(2,4-dichloro-3-thiénylamino)-1H-thiéno[3,4-d]imidazole,
et sels pharmaceutiquement acceptables et sels d'acide trifluoracétique de ceux-ci.

6. Composés de formule I ou II ou sels pharmaceutiquement acceptables de ceux-ci selon une ou plusieurs parmi les revendications 1 à 5, pour une utilisation comme médicament.

7. Utilisation d'un composé de formule I ou II ou de ses sels pharmaceutiquement acceptables selon une ou plusieurs parmi les revendications 1 à 5, pour la préparation d'un médicament destiné au traitement ou à la prophylaxie de troubles du dynamisme respiratoire, de troubles respiratoires liés au sommeil, des apnées du sommeil, ou du ronflement, pour le traitement ou pour la prophylaxie de troubles rénaux aigus et chroniques, d'insuffisance rénale aiguë ou d'insuffisance rénale chronique, ou d'un dysfonctionnement intestinal, ou destiné au traitement ou à la prophylaxie de l'hypertension artérielle, de l'hypertension essentielle, de troubles du système nerveux central, de troubles résultant d'une hyperexcitabilité du SNC, d'une épilepsie ou de convulsions centralement induites, et de troubles du système nerveux central, d'états d'anxiété, de dépressions et de psychoses, destiné au traitement ou à la prophylaxie de lésions aiguës ou chroniques, de troubles et de séquelles indirectes d'organes ou de tissus qui sont provoqués par des évènements ischémiques ou de reperfusion, d'arythmies, d'athérosclérose, d'hypercholestérolémie, de maladies dans lesquelles une prolifération cellulaire constitue une cause primaire ou secondaire, du cancer, de l'hypertrophie de la prostate et de l'hyperplasie de la prostate, de troubles fibrotiques d'organes internes, de l'insuffisance cardiaque ou de l'insuffisance cardiaque congestive, de thromboses, de troubles de la fonction biliaire, de l'infestation par des ectoparasites, de troubles résultant d'un dysfonctionnement endothélial, de la claudication intermittente, de troubles protozoaires, de la malaria, pour le traitement du choc, du diabète et de l'endommagement diabétique tardif, de troubles inflammatoires aigus ou chroniques ou pour la préparation d'un médicament destiné à la conservation et au stockage de greffes pour des mesures chirurgicales, pour la préparation d'un médicament destiné à une utilisation dans des opérations chirurgicales et des greffes d'organes, pour la préparation d'un médicament destiné à la prévention des modifications tissulaires liées à l'âge, pour la conservation de la santé et la prolongation de la vie, ou pour la préparation d'un médicament destiné à réduire les effets cytotoxiques ou pour une utilisation comme agent de diagnostic.

8. Utilisation d'un composé de formule I ou II ou de ses sels pharmaceutiquement acceptables selon une ou plusieurs parmi les revendications 1 à 5, en association avec un ou plusieurs autres médicaments ou ingrédients actifs pour la préparation d'un médicament destiné au traitement ou à la prophylaxie de troubles du dynamisme respiratoire, de troubles respiratoires liés au sommeil, des apnées du sommeil, ou du ronflement, pour le traitement ou pour la prophylaxie de troubles rénaux aigus et chroniques, d'insuffisance rénale aiguë ou d'insuffisance rénale chronique, ou d'un dysfonctionnement intestinal, ou destiné au traitement ou à la prophylaxie de l'hypertension artérielle, de l'hypertension essentielle, de troubles du système nerveux central, de troubles résultant d'une hyperexcitabilité du SNC, d'une épilepsie ou de convulsions centralement induites, et de troubles du système nerveux central, d'états d'anxiété, de dépressions et de psychoses, destiné au traitement ou à la prophylaxie de lésions aiguës ou chroniques, de troubles et de séquelles indirectes d'organes ou de tissus qui sont provoqués par des évènements ischémiques ou de reperfusion, d'arythmies, d'athérosclérose, d'hypercholestérolémie, de maladies dans lesquelles une prolifération cellulaire constitue une cause primaire ou secondaire, du cancer, de l'hypertrophie de la prostate et de l'hyperplasie de la prostate, de troubles fibrotiques d'organes internes, de l'insuffisance cardiaque ou de l'insuffisance cardiaque congestive, de thromboses, d'un dysfonctionnement biliaire, de l'infestation par des ectoparasites, de troubles résultant d'un dysfonctionnement endothélial, de la claudication intermittente, de troubles protozoaires, de la malaria, pour le traitement du choc, du diabète et de l'endommagement diabétique tardif, de troubles inflammatoires aigus ou chroniques ou pour la préparation d'un médicament destiné à la conservation et au stockage de greffes pour des mesures chirurgicales, pour la préparation d'un médicament destiné à une utilisation dans des opérations chirurgicales et des greffes d'organes, pour la préparation d'un médicament destiné à la prévention des modifications tissulaires liées à l'âge, pour la conservation de la santé et la prolongation de la vie, ou pour la préparation d'un médicament destiné à réduire les effets cytotoxiques.

9. Utilisation d'un composé de formule I ou II ou des sels pharmaceutiquement acceptables de celui-ci selon une ou plusieurs parmi les revendications 1 à 5, seul ou en association avec d'autres médicaments
ou ingrédients actifs, pour la préparation d'un médicament destiné au traitement ou à la prophylaxie de la détérioration du dynamisme respiratoire, des perturbations respiratoires liées au sommeil ou des apnées du sommeil.

10. Utilisation d'un composé de formule I ou II ou des sels pharmaceutiquement acceptables de celui-ci selon une ou plusieurs parmi les revendications 1 à 5, seul ou en association avec d'autres médicaments ou ingrédients actifs, pour la préparation d'un médicament destiné au traitement ou à la prophylaxie du ronflement.

11. Utilisation d'un composé de formule I ou II ou des sels pharmaceutiquement acceptables de celui-ci selon une ou plusieurs parmi les revendications 1 à 5, seul ou en association avec d'autres médicaments ou ingrédients actifs, pour la préparation d'un médicament destiné au traitement ou à la prophylaxie de troubles rénaux aigus et chroniques, d'insuffisance rénale aiguë ou d'insuffisance rénale chronique.

12. Utilisation d'un composé de formule I ou II ou des sels pharmaceutiquement acceptables de celui-ci selon une ou plusieurs parmi les revendications 1 à 5, seul ou en association avec d'autres médicaments ou ingrédients actifs, pour la préparation d'un médicament destiné au traitement ou à la prophylaxie d'un dysfonctionnement intestinal.

13. Composition curative destinée à une utilisation humaine, vétérinaire ou phytoprotectrice, comprenant une quantité efficace d'au moins un composé de formule I ou II et/ou des sels pharmaceutiquement acceptables de celui-ci selon une ou plusieurs parmi les revendications 1 à 5, conjointement avec des véhicules et des additifs pharmaceutiquement acceptables.

14. Composition curative destinée à une utilisation humaine, vétérinaire ou phytoprotectrice, comprenant une quantité efficace d'au moins un composé de formule I ou II et/ou des sels pharmaceutiquement acceptables de celui-ci selon une ou plusieurs parmi les revendications 1 à 5, conjointement avec des véhicules et des additifs pharmaceutiquement acceptables, en association avec d'autres ingrédients actifs pharmacologiques ou des médicaments.

15. Procédé de préparation d'un composé de formule 1 ou Il et/ou des sels pharmaceutiquement acceptables de celui-ci, **caracterisé en ce qu'**on procède
a) à la réaction d'une diamine de formule III ou IV avec un cyanate de formule V pour donner un dérivé d'urée de formule VI ou VII,
b) à la cyclisation du dérivé d'urée de formule VI ou VII pour donner un composé de formule la ou IIa,
et
c) pour préparer un composé de formule I ou II dans lesquelles R4 est autre qu'hydrogène, à la dérivatisation d'un composé de formule la ou IIa pour donner un composé de formule I ou II 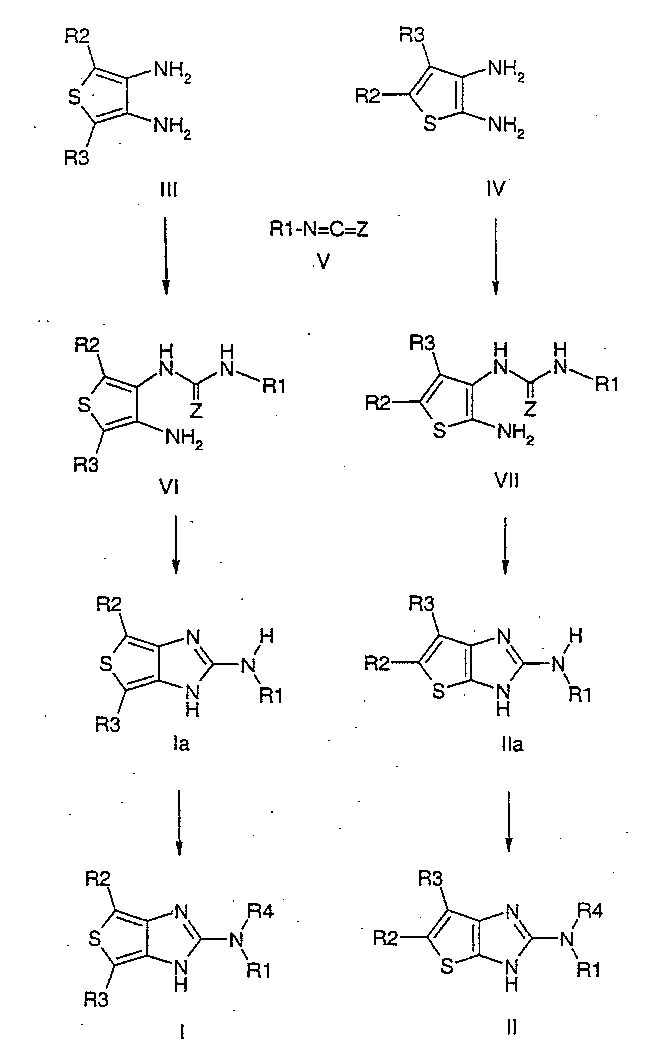
dans lesquelles R1, R2, R3 et R4 sont chacun tels que définis dans les revendications 1 à 5, et dans lesquelles Z est oxygène ou préférablement soufre.
